# EUROPÄISCHE PATENTANMELDUNG

(11) **EP 1 591 525 A2**
(43) Veröffentlichungstag der Anmeldung: **02.11.2005**
(21) Anmeldenummer: 05101190.6
(22) Anmeldetag: 17.02.2005
(51) Int. Cl.: C12N 15/11, C12N 15/63, A61K 48/00

(54) **Oligoribonukleotide zur Behandlung von irritativen und/oder entzündlichen Hauterscheinungen durch RNA-Interferenz**

(30) Priorität: 03.03.2004 DE 102004010547
(71) Anmelder: Beiersdorf AG, 20245 Hamburg (DE)
(72) Erfinder: Wolber, Rainer, 22399, Hamburg (DE); Mundt, Claudia, 28207, Bremen (DE); Gallinat, Stefan, 22880, Wedel (DE); Breitenbach, Ute, 20251, Hamburg (DE); Kolbe, Ludger, 21255, Dohren (DE)

(57) **Zusammenfassung**

Doppelsträngiges Oligoribonukleotid oder ein physiologisch verträgliches Salz davon, das in der Lage ist, den Abbau von mRNA von einem oder mehreren an der Entzündung und/oder Irritation der Haut beteiligten Strukturen zu induzieren.

## Beschreibung

Die Erfindung betrifft Oligoribonukleotide, die den Abbau von mRNA von am Entzündungsprozess der Haut beteiligten Enzymen und Strukturen induzieren und die sich insbesondere zur Prophylaxe und Behandlung von entzündlichen Hautzuständen und/oder zum Hautschutz bei empfindlich determinierter Haut eignen.
Darüber hinaus betrifft die Erfindung kosmetische oder dermatologische Formulierungen, kosmetische oder dermatologische Formulierungen, insbesondere kosmetische oder dermatologische Formulierungen, welche die Haut nach einem Sonnenbad oder einer Rasur gezielt pflegen und die Nachreaktionen der Haut auf die Einwirkung von UV-Strahlung und/oder die Reizung durch Rasur vermindern.

Die Haut, insbesondere die Epidermis, ist als Barriereorgan des menschlichen Organismus in besonderem Maße äußeren Einwirkungen unterworfen. Nach dem heutigen wissenschaftlichen Verständnis repräsentiert die Haut ein immunologisches Organ, das als immunkompetentes peripheres Kompartiment eine eigene Rolle in induktiven, effektiven und regulativen Immunprozessen des Gesamtorganismus spielt.

Die Epidermis ist reich mit Nerven und Nervenendapparaten wie Vater-Pacini-Lamellenkörpern, Merkel-Zell-Neuritenkomplexen und freien Nervenendigungen für Schmerz-, Kälte-, Wärmeempfindung und Juckreiz ausgestattet.

Bei Menschen mit sensibler, empfindlicher oder verletzlicher Haut kann ein mit "Stinging" (<engl.> "to sting" = verletzen, brennen, schmerzen) bezeichnetes neurosensorisches Phänomen beobachtet werden. Diese "sensible Haut" unterscheidet sich grundsätzlich von "trockener Haut" mit verdickten und verhärteten Hornschichten.

Typische Reaktionen des "Stinging" bei sensibler Haut sind Rötung, Spannen und Brennen der Haut sowie Juckreiz.

"Stinging"-Phänomene können als kosmetisch zu behandelnde Störungen angesehen werden. Starker Juckreiz dagegen, insbesondere bei Atopie auftretendes starkes Hautjucken sowie Juckreiz bei Hauterkrankungen, kann auch als schwerwiegendere dermatologische Störung bzw. neurosensorisches Phänomen bezeichnet werden.

Typische, mit den Begriffen "Stinging" oder "empfindlicher Haut" in Verbindung gebrachte, störende neurosensorische Phänomene sind Hautrötung, Kribbeln, Prickeln, Spannen und Brennen der Haut und Juckreiz. Sie können durch stimulierende Umgebungsbedingungen - z. B. Massage, Einwirkung (waschaktiver) Tenside, Wettereinfluss wie Sonne, Kälte, Trockenheit, aber auch feuchte Wärme, Wärmestrahlung und UV-Strahlung, z. B. der Sonne - hervorgerufen werden.

In Journal of the Society of Cosmetic Chemists" 28, S.197 - 209 (Mai 1977) beschreiben P. J. Frosch und A. M. Kligman eine Methode zur Abschätzung des "Stinging-Potentials" topisch verabreichter Substanzen. Als positive Substanzen werden hier z. B. Milchsäure und Brenztraubensäure eingesetzt. Bei Messung nach dieser Methode wurden aber auch Aminosäuren, insbesondere Glycin, als neurosensorisch aktiv ermittelt (solche Substanzen werden "Stinger" genannt).

Nach bisherigen Erkenntnissen tritt eine derartige Empfindlichkeit gegenüber ganz bestimmten Substanzen individuell unterschiedlich auf. Dies bedeutet, dass eine Person, die bei Kontakt mit einer Substanz "Stingingeffekte" erlebt, sie mit hoher Wahrscheinlichkeit bei jedem weiteren Kontakt wiederholt erleben wird. Der Kontakt mit anderen "Stingern" kann aber ebenso gut ohne jede Reaktion verlaufen.

Viele mehr oder weniger empfindliche Personen haben auch bei Verwendung mancher deodorierenden oder antitranspirierend wirkenden Zubereitungen unter erythematösen Hauterscheinungen zu leiden.

Erythematöse Hauterscheinungen treten ferner auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Es war also die Aufgabe der vorliegenden Erfindung, den Nachteilen des Standes der Technik abzuhelfen.

Insbesondere sollten Wirkstoffe und Zubereitungen, solche Wirkstoffe enthaltend, zur kosmetischen und dermatologischen Behandlung und/oder Prophylaxe erythematöser, entzündlicher, allergischer oder autoimmunreaktiver Erscheinungen, insbesondere Dermatosen, aber auch des Erscheinungsbildes des "Stingings" zur Verfügung gestellt werden.

Neben den positiven Auswirkungen des Sonnenlichtes, wie dem allgemeinen Wohlbefinden, der Bildung von Vitamin D3 und der Aknebehandlung, gibt es auch negative Auswirkungen, denen es entgegenzuwirken gilt.

Setzt man die Haut zu lange der Sonne oder einer künstlichen Strahlenquelle aus, so entwickelt sich nach einer Latenzzeit von 2 bis 3 Stunden eine gegen die unbestrahlte Haut stark abgegrenzte Hautrötung, das Erythema solare. Bei dem so entstehenden Sonnenbrand unterscheidet man zwischen
1. Grad: Erythem (Rötung, Wärmegefühl)
   klingt nach 2 bis 3 Tagen wieder ab und verschwindet unter gleichzeitig zunehmender Pigmentierung,
2. Grad: Blasenbildung
   auf der Haut bilden sich Blasen mit Brennen und Jucken, die Oberhaut wird flächig abgestoßen
3. Grad: Zellschädigung
   es treten tiefgehende Zellschädigungen auf, der Körper reagiert mit Fieber, die Oberhaut wird großflächig abgestoßen.
   Der 2. und 3. Grad werden auch als Dermatitis solare bezeichnet.

Die Bildung des Erythems ist abhängig von der Wellenlänge. Der Erythembereich des UV-B liegt zwischen 280 nm und 320 nm.

Etwa 90 % der auf die Erde gelangenden ultravioletten Strahlung besteht aus UV-A-Strahlen mit einer Wellenlänge zwischen 320 nm und 400 nm. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Man hat lange Zeit fälschlicherweise angenommen, dass die langwellige UV-A-Strahlung nur eine vernachlässigbare biologische Wirkung aufweist und dass dementsprechend die UV-B-Strahlen für die meisten Lichtschäden an der menschlichen Haut verantwortlich seien. Inzwischen ist allerdings durch zahlreiche Studien belegt, dass UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluss der UV-B-Strahlung durch UV-A-Strahlung noch verstärkt werden.

Da die Beiträge der verschiedenen Wellenlängebereiche des UV-Lichtes zu lichtbedingten Hautveränderungen nicht vollständig geklärt sind, geht man heute verstärkt davon aus, dass vorbeugender Schutz sowohl gegen UV-A- als auch gegen UV-B-Strahlen, beispielsweise durch Auftrag von Lichtschutzfiltersubstanzen in Form einer kosmetischen oder dermatologischen Formulierung auf die Haut, von grundsätzlicher Wichtigkeit ist. Kosmetische oder dermatologische Mittel sollen, in dünner Schicht auf die Haut aufgetragen, diese vor den negativen Auswirkungen der Sonnenstrahlung schützen.

Ein Sonnenbad wird von den meisten Menschen als angenehm empfunden, die nachteiligen Folgen zunächst nicht beachtet. Allerdings hat sich in den letzten Jahren durchaus ein Bewusstsein über die negativen Auswirkungen einer zu intensiven Sonnenbestrahlung herausgebildet, weshalb mehr und stärker schützende Sonnenschutzmittel angewendet werden.

Der Sonnenbrand bzw. das Lichterythem sind die akuten Erscheinungsformen der Lichteinwirkung. Neben den bereits beschriebenen Wirkungen der UV-Strahlen kommt es in der Nachreaktion der Haut ferner zu einer verminderten Sebumproduktion und einem Austrocknen der Haut. Zur Linderung und zur Pflege der lichtstrapazierten Haut können deshalb spezielle Wirkstoffe eingesetzt werden, wie beispielsweise Rückfettungs- und Feuchthaltemittel, entzündungslindernde und kühlende Stoffe, lokal a-naesthesierende Stoffe und/oder desinfizierende Stoffe, um mögliche Hautinfektionen zu verhindern.

Eingesetzt werden z. B. aus Pflanzen gewonnene entzündungslindernde bzw. -hemmende Wirkstoffe wie Azulen und Bisabolol (Kamille), Glycyrrhizin (Süßholzwurzel), Hamamelin (Hamamelis) oder Gesamtextrakte, z. B. aus Aloe vera oder Kamille. Diese zeigen bei leichteren Formen und lokal begrenzten Erythemreaktionen gewisse Erfolge. Gleiches gilt für Cremes mit einem hohen Gehalt an ätherischen Ölen oder Panthenol.

So genannte Aftersun-Präparate sind dazu bestimmt, die Haut nach dem Sonnenbad zu kühlen und ihr Feuchthaltevermögen zu verbessern, wobei die Vermittlung des Kühleffektes eine zentrale Rolle spielt. Dieser Kühleffekt wird in der Regel durch hohe Mengen an Ethanol erzielt, welches beim Verteilen der Formulierung auf der Haut spontan verdunstet.

Nachteil dieser Formulierungen des Standes der Technik ist allerdings, dass eine langfristige Regeneration der Haut durch bloße Kühlung nicht erreichbar ist.

Aufgabe der vorliegenden Erfindung war es also, kosmetische oder dermatologische Zubereitungen zu finden, die die Nachteile des Standes der Technik nicht aufweisen und die insbesondere lichtstrapazierte Haut lang anhaltend pflegen.

Der Wuchs des Barthaares wird beim heranwachsenden Mann durch die gesteigerte Bildung männlicher Hormone während der Pubertät ausgelöst. Hormonelle Störungen bei der Frau können ebenfalls zu einer Form von Bartwuchs führen, die allerdings in aller Regel in seiner Ausprägung deutlich hinter dem des männlichen Bartwuchses zurückbleibt.

Die Rasur des Gesichts oder anderer behaarter Körperteile (wie beispielsweise der Beine, der Achsel oder des Intimbereichs) kann durch mancherlei Zwänge - z. B. religiöser oder kultureller Art - motiviert werden; im einfachsten Fall ist der Haarwuchs für die betreffende Person schlichtweg aus kosmetischen Gründen unerwünscht.
Eine Rasur wird entweder trocken oder nass durchgeführt. Die Entwicklung von neuen mechanischen und elektrischen Nass- und Trockenrasiertechniken ermöglicht heutzutage ein sicheres und gründliches Entfernen des (Bart-) Haares. Bei der Nassrasur sind in der Regel chemische Hilfsmittel - beispielsweise in Form von Rasiergelen, -seifen oder -schäumen - unerlässlich. Diese werden benötigt, um das (Bart-) Haar aufweichen und so den zum Durchschneiden erforderlichen Kraftaufwand - und damit das unangenehme Ziehen am Haarschaft - zu minimieren. Das Aufweichen der (Bart-) Haare wird durch Wasseraufnahme erreicht, die durch die Erhöhung des pH-Werts der Haare ermöglicht wird. Nassrasurmittel enthalten deshalb in der Regel Seifen bzw. Fettsäuresalze, deren pH-Wert im Bereich von 8-10 liegt. Produkte für die Nassrasur erzeugen daher ein typisches Hautgefühl, welches nach der Anwendung auftritt. Die Haut fühlt sich trocken und rau an. Dieses Hautgefühl wird in der kosmetischen Fachwelt auch als "squeaky-feeling" bezeichnet und ist bei Verbrauchern und Verbraucherinnen äußerst unbeliebt.

Auch bei der Trockenrasur sind kosmetische Mittel häufig empfehlenswert, um eine möglichst dichte Rasur zu bewirken, d. h. das (Bart-) Haar möglichst nahe an der Hautoberfläche abzuschneiden.

Die von der Rasur betroffenen Hautpartien können allerdings nicht nur durch die Rasurhilfsmittel gereizt werden, auch die mechanische Reizung durch das Rasieren an sich stellt eine Belastung der Haut dar, welche ein unangenehmes Hautgefühl (den sog. "Rasurbrand") zur Folge haben kann.

Eine weitere Aufgabe der vorliegenden Erfindung war es daher, kosmetische oder dermatologische Zubereitungen zu finden, die die Nachreaktionen der Haut auf die (mechanische) Reizung durch das Rasieren besser vermindern.

Es war vielmehr überraschend und darin liegt die Lösung dieser Aufgaben, dass die Verwendung von kosmetischen oder dermatologischen Formulierungen mit einem Gehalt an interferierenden RNAs zur Pflege lichtstrapazierter und/oder rasurgestresster Haut sowie zur Linderung der Nachreaktionen der Haut auf die Einwirkung von UV-Strahlung und/oder auf die Reizung der Haut durch Rasieren den Nachteilen des Standes der Technik abhelfen würden.

Die Formulierungen im Sinne der vorliegenden Erfindung sind in jeglicher Hinsicht überaus befriedigende Präparate, die sich durch eine lang anhaltende Pflegewirkung auszeichnen. Es war für den Fachmann nicht vorauszusehen gewesen, dass die erfindungsgemäss verwendeten Formulierungen lichtstrapazierte und rasurgestresste Haut besser pflegen, die Nachreaktionen der Haut auf die Einwirkung von UV-Strahlung und auf die (mechanische) Reizung durch Rasieren besser vermindern, die vom Sonnenbaden und der Rasur gereizte Haut besser beruhigen, leichten Sonnenbrand und den Rasurbrand schneller zum Abklingen bringen, besser die Hautglättung fördern, sich durch bessere Pflegewirkung auszeichnen, bessere sensorische Eigenschaften, wie beispielsweise die Verteilbarkeit auf der Haut oder das Einzugsvermögen in die Haut, aufweisen würden
als die Zubereitungen des Standes der Technik.

Es war ferner erstaunlich, dass die Zubereitungen im Sinne der vorliegenden Erfindung die Nachreaktionen der Haut auf die Einwirkung von UV-Strahlung und auf die (mechanische) Reizung durch Rasieren auch dann vermindern, wenn sie bereits vor bzw. während eines Sonnenbades und/oder einer Rasur verwendet (d. h. auf die Haut aufgebracht) werden.

Die Erfindung ist selbstverständlich nicht auf Zubereitungen beschränkt, welche nach dem Sonnenbad und/oder der Rasur angewendet werden, sondern umfasst naturgemäss alle kosmetischen und dermatologischen Anwendungen, bei welchen eine stresslindernde Wirkung gewünscht oder von Vorteil sein könnte. Das oben Gesagte zur positiven Wirkung der erfindungsgemäss verwendeten Formulierungen gilt in gleicher Weise bei lichtstrapazierter Haut wie auch bei rasurstrapazierter Haut.

Die oben beschriebenen Reizungen der Haut durch UV-Licht, Rasur oder auch der sensible Hautzustand an sich werden u.a. durch so genannte Entzündungsmediatoren vermittelt. Hier sind vor allem zwei große Gruppen zu nennen, die ArachidonsäureMetaboliten und pro-entzündliche Cytokine. Diese alle werden als an der Entzündung und/oder Irritation der Haut beteiligte Strukturen im Sinne dieser Schrift bezeichnet. Arachidonsäure ist eine mehrfach ungesättigte, langkettige Fettsäure (20:4), die bei Reizung durch die Aktivierung einer Phospholipase A2 aus Membranen freigesetzt wird. Sie dient dann als Substrat für Cyclooxygenasen (COX) oder Lipoxygenasen (LOX). Durch die Aktion weiterer nachgeschalteter Enzyme kommt es letztlich zur Freisetzung von Prostaglandinen und Leukotrienen (Zusammen als Eicosanoide bezeichnet) die über spezifische Rezeptoren auf den Zielzellen diese aktivieren. In der Haut lösen sowohl Prostaglandine als auch Leukotriene Entzündungsreaktionen wie Erytheme, Ödeme, Einwanderung von Entzündungszellen und gesteigerte Schmerzwahrnehmung. Zur Inhibierung der Prostaglandinfreisetzung gibt es eine Fülle von Medikamenten, am bekanntesten das Aspirin, die aber alle an der gleichen Stelle angreifen, den Cyclooxygenasen. Da die COX-1 aber auch wichtige regulatorische Funktionen im Magen und den Nieren hat, ist es wichtig präferentiell die COX-2 zu inhibieren, ein Enzym, dass durch inflammatorische Stimuli induziert wird. Pro-entzündliche Leukotriene werden durch die 5-Lipoxygenase gebildet, einem Enzym, das durch externe Stimuli über das Protein FLAP (Five Lipoxygenase Aktivating Protein) aktiviert werden kann. Lipoxygenase Inhibitoren sind noch auf einem experimentellen Stadium, da es eine Reihe weiterer Lipoxygenasen gibt, die teilweise auch eher eine anti-inflammatorische Wirkung haben, ist es sehr wichtig Inhibitoren zu entwickeln die eine hohen Selektivität für die LOX-5 haben.
Die zweite große Gruppe der pro-entzündlichen Proteine sind die Cytokine, Proteine die autokrin oder parakrin Zellen aktivieren und damit Abwehr- oder Schutzfunktionen auslösen oder ganz einfach chemotaktisch wirken. Für die Haut besonders wichtig sind in diesem Zusammenhang Interleukin-1 alpha und beta (IL-1α, IL-1β) das teilweise konstitutiv in den Hautzellen gebildet wird (IL-1a) oder auf einen Reiz in gebildet (IL-1β) oder verstärkt exprimiert wird (IL-1a), Interleukin-6 (IL-6) das in Folge irritativer Reize exprimiert wird, dem Interleukin-8 (IL-8), das bei Hautirritationen freigesetzt wird und dann Entzündungszellen anlockt (und somit eigentlich ein Chemokin ist) sowie Tumornekrose Faktor alpha (TNF-α), welches bei Entzündungen verstärkende aber auch proapoptotische Wirkungen haben kann. In der Therapie entzündlicher Erkrankungen werden zur Unterdrückung pro-entzündlicher Cytokine vor allem Kortikosteroide eingesetzt, mit den bekannten Nebenwirkungen.

Die bisher therapeutisch eingesetzten Inhibitoren der Prostaglandinproduktion sind allesamt Inhibitoren der Enzymaktivität. Wirkstoffe, welche die Expression der Enzyme inhibieren sind bisher noch in der experimentellen Erprobung. Dieser Weg sowie die Induktion natürlicher Antagonisten (z.B. Interleukin-1 Rezeptorantagonist, IL-1RA) wird zur Verminderung pro-entzündlicher Cytokine intensiv erforscht. Zumeist handelt es sich bei den dabei untersuchten Wirkstoffen um Substanzen, die mit der Signaltransduktionskette interferieren und damit letztlich die Expression der Proteine unterdrücken.

Je nach dem, wo in die Signaltransduktion eingegriffen wird, und wie spezifisch das gelingt, erkauft man sich den Effekt mit einer Reihe von mehr oder weniger großen Nachteilen, da ungewollt auch andere Wege der Signalkaskade blockiert werden. Diese Effekte sind zumindest für einen Teil der erheblichen Nebenwirkungen der herkömmlichen Therapie mit Kortikosteroide verantwortlich.

Eine Therapie über RNA-Interferenz, bei der ganz gezielt nur das Zielmolekül in seiner Expression inhibiert würde hätte deshalb erhebliche Vorteile.

Fire et al., Trends Genet. 15 (1999) 358-363 haben gezeigt, dass sich die Genexpression posttranskriptional durch die Anwesenheit doppelsträngiger RNA-Fragmente (dsRNA), die homolog zur Sequenz der mRNA des untersuchten Gens ist, inhibieren lässt, und diesen Vorgang als RNA-Interferenz (RNAi) bezeichnet. Die dsRNA bewirkt auf noch ungeklärte Weise den spezifischen Abbau der homologen mRNA in der Zelle und verhindert so die Proteinproduktion.

Die WO01/29058 offenbart die Identifikation von Genen, die an der RNAi beteiligt sind, sowie deren Verwendung zur Modulation des RNAi-Aktivität.

Elbashir et al., Nature 411 (2001) 494-498, beschreiben die spezifische Inhibierung der Expression von endogenen und heterologen Genen in verschiedenen Säugerzellen durch kurze, interferierende RNAs (short interfering RNAs, siRNAs). Es wurden doppelsträngige RNA-Fragmente mit einer Länge von 21 Nukleotiden eingesetzt.

Aus der WO01/68836 ist die Verminderung der Genexpression in Zellen durch dsRNA bekannt. Die dsRNA enthält eine Nukleotidsequenz, die unter den physiologischen Bedingungen der Zelle mit der Nukleotidsequenz zumindest eines Teils des zu inhibierenden Gens hybridisiert. Die dsRNA weist vorzugsweise eine Länge von 400 bis 800 Nukleotiden auf.

Die WO01/75164 offenbart die Verwendung von dsRNA mit einer Länge von 21 bis 23 Nukleotiden zur spezifischen Inaktivierung von Genfunktionen in Säugerzellen durch RNAi.

Brummelkamp et al., Science 296 (2002) 550-553, beschreiben ein Vektorsystem, das die Synthese von siRNAs in Säugerzellen auslösen und so die Genexpression eines Zielgens inhibieren soll.

Die EP 1 214 945 A2 offenbart die Verwendung von dsRNA mit einer Länge von 15 bis 49 Basenpaaren zur Hemmung der Expression eines vorgegebenen Zielgens in Säugerzellen. Die dsRNA kann zur Erhöhung ihrer Stabilität modifiziert sein und soll die Behandlung von Krebs, viralen Erkrankungen und Morbus Alzheimer erlauben.

Die WO02/053773 betrifft ein *in vitro* Verfahren zur Bestimmung des Hautstress und der Hautalterung bei Menschen und Tieren, zur Durchführung des Verfahrens geeignete Test-Kitts und Biochips sowie ein Testverfahren zum Nachweis der Wirksamkeit von kosmetischen oder pharmazeutischen Wirkstoffen gegen Hautstress und Hautalterung.

Oligoribonukleotide, die sich zur Prophylaxe und Behandlung von unerwünschten Entzündungen/Irritationen der Haut eignen, wurde bisher nicht beschrieben.

Eine Aufgabe der vorliegenden Erfindung ist die Bereitstellung von Zusammensetzungen, die eine wirksame Behandlung von und Prophylaxe vor Irritationen, Entzündungen und empfindlichen Hautzuständen, insbesondere gegen Sonnenbrand und Rasurbrand ermöglichen, ohne die Nachteile des Standes der Technik zu zeigen.

Nachfolgend werden die an der Entzündung der Haut beteiligten Enzyme und Proteine auch zusammenfassend "pro-entzündliche Proteine" genannt.

Diese Aufgabe wird durch Oligoribonukleotide gelöst, die in der Lage sind, die Expression der Gene von Enzymen und Proteinen zu inhibieren, die am Entzündungsprozess der Haut beteiligt sind, insbesondere durch doppelsträngige Oligoribonukleotide oder physiologisch verträgliche Salze davon, die in der Lage sind, den Abbau von mRNA von einem oder mehreren an der Entzündung und/oder Irritation der Haut beteiligten Strukturen, insbesondere an der Produktion von pro-entzündlichen Eicosanoiden oder Cytokinen beteiligte Strukturen, zu induzieren.

Unter Eicosanoid-produzierende Enzyme werden in erster Linie die an der Eicosanoidsynthese beteiligten Enzyme (COX-2, LOX-5) verstanden.

Neben den genannten Oligoribonukleotiden sind erfindungsgemäss auch physiologisch verträgliche Salze solcher Oligoribonukleotide geeignet. Im folgenden wird der Einfachheit halber der Begriff Oligoribonukleotid sowohl für die Oligoribonukleotide selbst als auch für deren Salze verwendet, wenn nicht anders angegeben. Der Begriff Oligoribonukleotid schließt auch modifizierte Oligoribonukleotide ein.

Die bevorzugten an der Eicosanoidsynthese beteiligten Enzyme sind folgende Oxygenasen:
Prostaglandin G/H Synthase-2 PGH2 HUMAN (P35354)
   Prostaglandin G/H synthase 2 precursor (EC 1.14.99.1) (Cyclooxygenase -2) (COX-2) (Prostaglandin-endoperoxide synthase 2) (Prostaglandin H2 synthase 2) (PGH synthase 2) (PGHS-2) (PHS II). {GENE: PTGS2 OR COX2}
5-Lipoxygenase LOX5 HUMAN (P09917)
   Arachidonate 5-lipoxygenase (EC 1.13.11.34) (5-lipoxygenase) (5-LO). {GENE: ALOX5 OR LOG5} - Homo sapiens (Human)
5-Lipoxygenase activating Protein FLAP HUMAN (P20292)
   5-lipoxygenase activating protein (FLAP) (MK-886-binding protein). {GENE: ALOX5AP OR FLAP}

Bei der Prostaglandin G/H Synthase (PGHS-2) handelt es sich um das Schrittmacherenzym der Prostaglandin-Synthese. Das Enzym 5-Lipoxygenase ist das Schrittmacherenzym der Leukotrien-Synthese, FLAP aktiviert die Lipoxygenase und ist deshalb wichtig für die Leukotrienbildung. Bei den angegebenen Zahlen handelt es sich um die Zugangsnummern (Accession Numbers) der Swiss-PROT Datenbank des EMBL-EBI (European Bioinformatics Institute Heidelberg).

Zu den weiterhin bevorzugten Strukturen, die die Irritation und Entzündung der Haut beeinflussen, gehören pro-entzündliche Cytokine, insbesondere die folgenden:
Interleukin-1a 1A HUMAN (P01583)
   Interleukin-1 alpha precursor (IL-1 alpha) (Hematopoietin-1). {GENE: IL1A}
Interleukin-1β IL 1B HUMAN (P01584)
   Interleukin-1 beta precursor (IL-1 beta) (Catabolin). {GENE: IL1B}
Interleukin-6 IL6 HUMAN (P05231)
   Interleukin-6 precursor (IL-6) (B-cell stimulatory factor 2) (BSF-2) (Interferon beta-2) (Hybridoma growth factor). {GENE: IL6 OR IFNB2}
Interleukin-8 IL8 HUMAN (P10145)
   Interleukin-8 precursor (IL-8) (CXCL8) (Monocyte-derived neutrophil chemotactic factor) (MDNCF) (T-cell chemotactic factor) (Neutrophil-activating protein 1) (NAP-1) (Lymphocyte-derived neutrophil-activating factor) (LYNAP) (Protein 3-10C) (Neutrophil-activating factor) (NAF) (Granulocyte chemotactic protein 1) (GCP-1) (Emoctakin). {GENE: IL8}
T Tumor Nekrose Factor -α TNFA HUMAN (P01375)
   Tumor necrosis factor precursor (TNF-alpha) (Tumor necrosis factor ligand superfamily member 2) (TNF-a) (Cachectin). {GENE: TNF OR TNFSF2 OR TNFA}

Angegeben sind hier die Zugangsnummern (Accession Numbers) der Swiss-PROT Datenbank des EMBL-EBI (European Bioinformatics Institute Heidelberg).

Ganz besonders bevorzugt sind Oligoribonukleotide, die die Expression der Cyclooxygenase, COX-2 inhibieren können.

In gleicher Weise besonders bevorzugt sind Oligoribonukleotide, die die Expression der Interleukin 1 α und β unterbinden.

Ebenso bevorzugt sind Oligoribonukleotide, die die Expression der mRNA der Interleukine 6 und 8 inhibieren können.

Bei den erfindungsgemässen Oligoribonukleotiden handelt es sich um RNA-Moleküle (RNAs), die die Expression dieser Enzyme ganz oder teilweise unterdrücken (Genabschaltung, Genesilencing), was vermutlich auf den Abbau der mRNA von einem der oben genannten Enzyme zurückzuführen ist. Dieser Vorgang wird als RNA-Interferenz (RNAi) bezeichnet. Die Erfindung betrifft somit Oligoribonukleotide, die den Abbau der mRNA von an der Irritation/Entzündung der Haut beteiligten Strukturen induzieren können. Die mRNA, deren Abbau bewirkt werden soll, wird im Folgenden auch Ziel-mRNA oder auch Zielsequenz genannt. Entsprechend wir unter Zielgen das Gen und insbesondere der codierende Bereich des Gens verstanden, dessen Expression ganz oder teilweise unterdrückt wird. Wenn nicht anders angegeben bezieht sich der Begriff Zielsequenz sowohl auf das Zielgen als auch auf die Ziel-mRNA. Der Abbau der mRNA von an der Irritation/Entzündung der Haut beteiligten Strukturen durch RNAi verläuft sequenzspezifisch, d.h. ein Oligoribonukleotid inhibiert in der Regel nur die Expression des korrespondierenden Zielgens.
Bevorzugt ist es, wenn das Oligoribonukleotid die Expression des Gens der an der Entzündung und/oder Irritation der Haut beteiligten Struktur um mindestens 30 %, besonders bevorzugt um 50% inhibiert.

Als Zielsequenz für die erfindungsgemässen Oligoribonukleotide sind die codierenden Bereiche (cDNA) der jeweiligen Gene bevorzugt, einschließlich der 5'- und 3'-UTR-Bereiche. Besonders bevozugt sind die Regionen der codierenden Bereiche, die 50 bis 100 Nukleotide stromabwärts des Startcodons liegen.

Die erfindungsgemässen Oligoribonukleotide stellen vorzugsweise doppelsträngige RNA-Moleküle (dsRNAs) dar, die zur Sequenz des Zielgens, bzw. einem Abschnitt davon, homolog sind, d.h. hinsichtlich Sense- und Antisense-Strang mit dem Zielgen übereinstimmen.

Homologie ist erfindungsgemäss auch dann gegeben, wenn die dsRNA nicht vollständig mit der Zielsequenz identisch ist. Die erfindungsgemässen Oligoribonukleotide weisen bezogen auf eine Länge von 20 Basenpaaren vorzugsweise maximal 0 bis 2, besonders bevorzugt 0 bis 1 und ganz besonders bevorzugt keine Abweichungen von der Zielsequenz auf, d.h. es sind maximal 0 bis 2 und insbesondere maximal 0 bis 1 Basenpaare gegen andere Basenpaare ausgetauscht.

Die erfindungsgemässen Oligoribonukleotide haben vorzugsweise eine Länge von 15 bis 49 Nukleotiden, vorzugsweise 17 bis 30, besonders bevorzugt 19 bis 25 und ganz besonders bevorzugt von 20 bis 23 Nukleotiden.

Gegenstand der Erfindung sind jedoch auch längere Nukleotidfragmente, wie z.B. dsRNAs, die in Ihrer Länge den jeweiligen Ziel-mRNAs bzw. cDNAs entsprechen. Diese können z.B. durch löslichen Drosophila Embryo Extrakt in Fragmente einer Länge von 21 bis 23 Nukleotiden überführt werden (vgl. WO01/75164). Langkettige dsRNA wird zudem intrazellulär zu kurzen Stücken abgebaut. Allerdings ist die direkte Verwendung langkettiger dsRNA im allgemeinen nicht bevorzugt, da diese in Säugerzellen eine u n-spezifische Inhibition der Translation bewirken kann.

Die erfindungsgemässen RNA-Duplexe können glatte (blunt ends) oder überstehende (sticky ends) Enden aufweisen. Als besonders wirksam haben sich doppelsträngige Oligoribonukleotide erwiesen, die am 3'-Ende von jedem Strang einen Überhang von 1 bis 6, vorzugsweise 1 oder 2 Nukleotiden aufweisen. Bei den überstehenden Nukleotiden handelt es sich vorzugsweise um 2'-Desoxynukleotide, besonders bevorzugt 2'-Desoxythymidinreste. Durch die Verwendung der 2'-Desoxynukleotide lassen sich die Kosten der RNA-Synthese reduzieren und die Widerstandsfähigkeit der RNA gegenüber dem Nukleaseabbau erhöhen. Die überstehenden Nukleotide müssen nicht zwangsläufig die zur Zielsequenz homologen Nukleotide sein und bleiben daher bei den oben definierten Abweichungen von der Zielsequenz unberücksichtig. Bevorzugt sind allerdings Oligoribonukleotide mit kurzen Überständen, insbesondere von 2 Nukleotiden, bei denen die überstehenden Nukleotide des antisense-Strangs der dsRNA zur Zielsequenz komplementär sind.

Als besonders wirksam haben sich Oligoribonukleotide erwiesen, die zu einem solchen Abschnitt des Zielgens und insbesondere der entsprechenden doppelsträngigen cDNA homolog sind, dessen sense-Strang 5'-seitig durch zwei Adenosinreste (A) und 3'-seitig durch zwei Thymidinreste (T) oder einen Thymidin- und einen Cytidinrest (C) begrenzt wird. Der durch AA und TT bzw. AA und TC begrenzte Abschnitt weist vorzugsweise eine Länge von 19 bis 21, insbesondere 19 Nukleotiden auf und hat demnach die allgemeine Form AA(N₁₉₋₂₁)TT oder AA(N₁₉₋₂₁)TC, wobei N für ein Nukleotid steht. Weiter bevorzugt sind Oligoribonukleotide, die zu einem Abschnitt des Zielgens bzw. der entsprechenden doppelsträngigen cDNA komplementär sind, der die allgemeine Form AA(N₁₉) bis AA(N₂₁) hat. Hierbei sind Oligoribonukleotide, die zu dem N₁₉₋₂₁-Fragment der genannte Bereiche homolog sind, besonders bevorzugt. Die besonders bevorzugten Oligoribonukleotide weisen somit eine Länge von 19 bis 21 Basenpaaren auf, wobei die diese Oligoribonukleotide bildenden Einzelstränge 3'-seitig vorzugsweise jeweils zwei zusätzliche 2'-Desoxynukleotide, insbesondere zwei 2'-Desoxythymidinreste aufweisen, so dass die dsRNA 19 bis 21 Basenpaare und pro Strang zwei überstehende 2'-Desoxynukleotide umfaßt.

Sollte das Zielgen keinen Bereich der Form AA(N₁₉₋₂₁) enhalten, wird nach Bereichen der Form NA(N₁₉₋₂₁) oder einem beliebigen Fragment der Form N₁₉₋₂₁ g esucht. N₁₉₋₂₁-Fragmente, die z.B. von AA und TT begrenzt werden, sind zwar bevorzugt, grundsätzlich sind erfindungsgemäss jedoch alle dsRNA-Fragmente geeignet, die zu der Zielsequenz homolog sind.

Die Figur 1 zeigt die einzelsträngige cDNA der Cycloxigenase-2 in der alle Fragmente der Form AA-N₁₉-TT und AA-N₁₉-TC optisch hervorgehoben sind. Da sich manche dieser Sequenzen überlappen sind die hervorgehobenen Bereiche teilweise beträchtlich länger als die einzelnen Fragmente. In Figur 2 sind diese Fragmente (targeted region) zusammen mit den entsprechenden homologen (senseRNA) und komplementären (antisenseRNA) RNA-Einzelsträngen dargestellt. Gezeigt sind einzelsträngige RNAs, die 3'-seitig durch zwei Desoxythymidinreste (dt) modifiziert sind. Die Hybridisierung von zwei komplementären einzelsträngigen RNAs ergibt dsRNA mit überstehenden 3'-Enden, die durch jeweils zwei 2'-Desoxythymidinreste gebildet werden.

Das Gen der Cycloxigenase-2 gehört zu den bevorzugten Zielgenen für die erfindungsgemässen Oligoribonukleotide. Oligoribonukleotide, die zu der von in Figur 1 gezeigten Sequenz abgeleiteten doppelsträngigen Sequenz, Abschnitten davon und insbesondere zu den doppelsträngigen Sequenzen, die von den in Figur 1 hervorgehobenen Abschnitten abgeleitet sind, homolog sind, sind demgemäss erfindungsgemäss besonders bevorzugt. Unter der von der in Figur 1 gezeigten Sequenz abgeleiteten doppelsträngigen Sequenz wird die Sequenz verstanden, die aus der in Figur 1 gezeigten Sequenz und dem dazu komplementären Strang gebildet wird. Die anderen Angaben sind entsprechend zu verstehen.
In Figur 3 ist die einzelsträngige cDNA der 5-Lipoxygenase (5-LOX) zu sehen. Auch hier sind die bevorzugten Sequenzbereiche, d.h. Sequenzbereiche mit einer Länge von 19 Nukleotiden, die durch AA und TT bzw. TC flankiert werden, hervorgehoben. Oligoribonukleotide, die zu der von der in Figur 3 gezeigten Sequenz abgeleiteten doppelsträngigen Sequenz, Abschnitten davon und insbesondere zu der doppelsträngigen Sequenz, die von dem in Figur 3 hervorgehobenen Bereich abgeleitet ist, homolog sind, sind erfindungsgemäss ebenfalls bevorzugt.

Figur 4 zeigt die einzelsträngige cDNA des Interleukins-6 (IL-6), wobei bevorzugte Sequenzbereiche wiederum markiert sind. Oligoribonukleotide, die zu der in Figur 4 hervorgehobenen abgeleiteten doppelsträngigen Sequenz, Abschnitten davon und insbesondere zu der doppelsträngigen Sequenz, die von den in Figur 4 hervorgehobenen Bereichen abgeleitet ist, homolog sind, sind erfindungsgemäss ebenfalls bevorzugt.

Die Figur 5 zeigt die einzelsträngige cDNA des Interleukins-1 alpha (IL-1α), wobei bevorzugte Sequenzbereiche gleichfalls markiert sind. Oligoribonukleotide, die zu der von der in Figur 5 gezeigten Sequenz abgeleiteten doppelsträngigen Sequenz, Abschnitten davon und insbesondere zu der doppelsträngigen Sequenz, die von den in Figur 5 hervorgehobenen Bereichen abgeleitet ist, homolog sind, sind erfindungsgemäss ebenfalls bevorzugt.

In Figur 6 ist die einzelsträngige cDNA des Interleukins-1 beta (IL-1β) zu sehen, wobei bevorzugte Sequenzbereiche wiederum markiert sind. Oligoribonukleotide, die zu der von der in Figur 6 gezeigten Sequenz abgeleiteten doppelsträngigen Sequenz, Abschnitten davon und insbesondere zu der doppelsträngigen Sequenz, die von den in Figur 6 hervorgehobenen Bereichen abgeleitet ist, homolog sind, sind erfindungsgemäss ebenfalls bevorzugt.

Die Figur 7 zeigt die einzelsträngige cDNA des Interleukins-8 (IL-8), wobei bevorzugte Sequenzbereiche gleichfalls markiert sind. Oligoribonukleotide, die zu der von der in Figur 7 gezeigten Sequenz abgeleiteten doppelsträngigen Sequenz, Abschnitten davon und insbesondere zu der doppelsträngigen Sequenz, die von den in Figur 7 hervorgehobenen Bereichen abgeleitet ist, homolog sind, sind erfindungsgemäss ebenfalls bevorzugt.

Figur 8 zeigt die einzelsträngige cDNA des 5-Lipoxygenase aktivierenden Proteins (FLAP), wobei bevorzugte Sequenzbereiche wiederum markiert sind. Oligoribonukleotide, die zu der von der in Figur 8 gezeigten Sequenz abgeleiteten doppelsträngigen Sequenz, Abschnitten davon und insbesondere zu der doppelsträngigen Sequenz, die von den in Figur 8 hervorgehobenen Bereichen abgeleitet ist, homolog sind, sind erfindungsgemäss ebenfalls bevorzugt.

In Figur 9 ist die einzelsträngige cDNA des Tumor Nekrose Faktor alpha (TNF-α) zu sehen, wobei bevorzugte Sequenzbereiche wiederum markiert sind. Oligoribonukleotide, die zu der von der in Figur 9 gezeigten Sequenz abgeleiteten doppelsträngigen Sequenz, Abschnitten davon und insbesondere zu der doppelsträngigen Sequenz, die von den in Figur 9 hervorgehobenen Bereichen abgeleitet ist, homolog sind, sind erfindungsgemäss ebenfalls bevorzugt.

Die erfindungsgemässen Oligoribonukleotide könnten vorteilhaft auch in Expressionsvektoren integriert werden, insbesondere solchen, die eine Expression der Oligoribonukleotide in Säugerzellen bewirken. Auf diese Weise läßt sich selbst bei einem intrazellulären Abbau der Oligoribonukleotide eine stabile Inhibierung der Expression des Zielgens erreichen, da durch die vektorgestützte Synthese ständig Oligoribonukleotide nachgeliefert werden. In einen Vektor können eine oder mehrere Kopien einer dsRNA integriert werden, aber auch jeweils eine oder mehrere Kopien von zwei oder mehr unterschiedlichen dsRNAs. Geeignete Vektorsysteme werden z.B. von Brummelkamp et al., a.a.O. beschrieben. Bevorzugt sind Säuger-Expressionsvektoren, insbesondere solche, die einen Polymerase III H1-RNA-Promotor und 5 bis 9 sogenannte Loops, die aus einer erfindungsgemässen dsRNA und einer gleichlangen Sequenz, die zur der erfindungsgemässen dsRNA revers komplementär ist und als Spacer dient, gebildet werden, und ein Terminationssignal von 5 aufeinanderfolgenden Thymidinresten enthalten. Die Vektoren enthalten somit 5 bis 9 Kopien des jeweiligen dsRNA-Moleküls. Hierbei kann es sich dsRNAs handeln, die für 1 Zielgen spezifisch sind, oder um dsRNAs, die für mehrere unterschiedliche Ziel gene spezifisch sind.

Die erfindungsgemässen Oligoribonukleotide können in Form der unmodifizierten Oligoribonukleotide vorliegen. Vorzugsweise handelt es sich jedoch um Oligoribonukleotide, die auf der Ebene der Zuckerreste, der Nukleobasen, der Phosphatgruppen und/oder des dazwischen befindlichen Skeletts chemisch modifiziert sein, um beispielsweise die Stabilität der Oligoribonukleotide in kosmetischen oder dermatologischen Zubereitungen und/oder in der Haut zu erhöhen, z.B. gegenüber einem nukleolytischem Abbau, um die Penetration der Oligoribonukleotide in die Haut und die Zelle zu verbessern, um die Wirksamkeit der Oligoribonukleotide günstig zu beeinflussen und/oder die Affinität zu den zu hybridisierenden Sequenzabschnitten zu verbessern.

Bevorzugt sind Oligoribonukleotide, bei denen eine oder mehrere Phosphatgruppen durch Phosphothioat-, Methylphosphonat- und/oder Phosphoramidatgruppen, wie z.B. N3'→P5'-Phosphoramidatgruppen, ausgetauscht sind. Besonders bevorzugt sind Oligoribonukleotide bei denen Phosphatgruppen durch Phosphothioatgruppen ausgetauscht sind. Es können eine oder mehrere der Phosphatgruppen des Oligoribonukleotids modifiziert sein. Bei einer teilweisen Modifikation werden vorzugsweise endständige Gruppen modifiziert, Oligoribonukleotide bei denen alle Phosphatgruppen modifiziert sind, sind jedoch besonders bevorzugt. Dies gilt sinngemäss auch für die im folgenden beschriebenen Modifikationen.

Bevorzugte Zuckermodifikationen umfassen den Austausch einer oder mehrerer Ribosereste des Oligoribonukleotids durch Morpholinringe (Morpholin-Oligoribonukleotide) oder durch Aminosäuren (Peptid-Oligoribonukleotide). Vorzugsweise sind sämtliche Ribosereste des Oligoribonukleotids gegen Aminosäurereste und insbesondere Morpholinreste ausgetauscht.

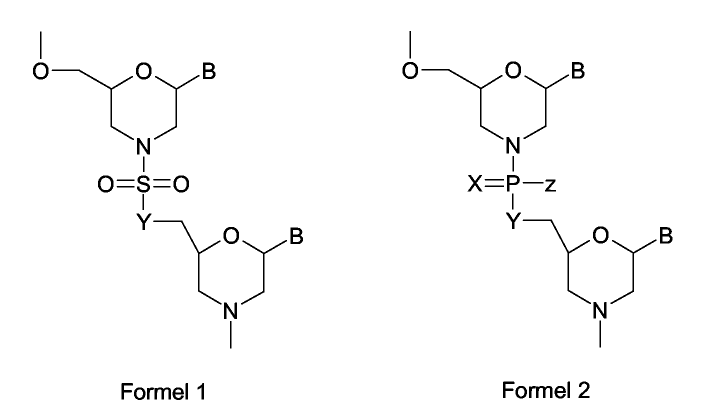

Besonders bevorzugt sind Morpholin-Oligoribonukleotide bei denen die Morpholinreste über Sulfonyl- oder vorzugsweise Phosphorylgruppen miteinander verbunden sind, wie in Formel 1 oder 2 zu sehen ist:

B steht für eine modifizierte oder nicht modifizierte Purin- oder Pyrimidinbase, vorzugsweise für Adenin, Cytosin, Guanin, oder Uracil,
X steht für O oder S, vorzugsweise O,
Y steht für O oder N-CH₃, vorzugsweise O,
Z steht für Alkyl, O-Alkyl, S-Alkyl, NH₂, NH(Alkyl), NH(O-Alkyl), N(Alkyl)₂, N(Alkyl)(O-Alkyl), vorzugsweise N(Alkyl)₂, wobei Alkyl für lineare oder verzweigte Alkylgruppen mit 1 bis 6 vorzugsweise 1 bis 3 und besonders bevorzugt 1 oder 2 Kohlenstoffatomen steht.

Die Formeln 1 und 2 stellen jeweils nur einen Ausschnitt aus einer Oligoribonukleotidkette dar.

Ganz besonders bevorzugt sind Morpholin-Oligoribonukleotide bei denen die Morpholinreste über Phosphorylgruppen miteinander verbunden sind, wie in Formel 2 gezeigt ist, bei denen X für O, Y für O und Z für N(CH₃)₂ steht.

Weiterhin können die Ribosereste durch Amino-, wie NH₂, Fluor, Alkyl oder O-Alkylreste, wie OCH₃, modifiziert werden, wobei 2'-modifizierte Oligoribonukleotide besonders bevorzugt sind. Beispielhafte Modifikationen sind 2'-Fluoro-, 2'-Alkyl-, 2'-O-Alkyl-, 2'-O-Methoxyethyl-Modifikationen, 5'-Palmitat-Derivate und 2'-O-Methylribonukleotide.

Die Modifizierung der Nukleotide von dsRNA wirkt in der Zelle einer Aktivierung der Proteinkinase PKR entgegen, die von doppelsträngiger RNA abhängig ist. Hierdurch wird eine unspezifische Inhibition der Translation vermieden. Zu diesem Zweck eignet sich insbesondere die Substitution mindestens einer 2'-Hydroxylgruppe der Nukleotide der dsRNA durch eine 2'-Amino- oder eine 2'-Methylgruppe. Weiterhin kann mindestens ein Nukleotid in mindestens einem Strang der dsRNA durch ein sogenanntes "locked nucleotide" ersetzt sein, das einen chemisch modifizierten Zuckerring enthält. Eine bevorzugte Modifikation des Zuckerrings ist eine 2'-O, 4'-C-Methylenbrücke. dsRNA, die mehrere "locked nucleotides" enthält, ist bevorzugt.

Wenn nicht anders angegeben, steht Alkyl hierin vorzugsweise für lineare, verzweigte oder cyclische Alkylgruppen mit 1 bis 30, vorzugsweise 1 bis 20, besonders bevorzugt 1 bis 10 und ganz besonders bevorzugt 1 bis 6 Kohlenstoffatomen. Verzweigte und cyclische Reste weisen naturgemäss mindestens 3 Kohlenstoffatome auf, wobei cyclische Reste mit mindestens 5 und insbesondere mindestens 6 Kohlenstoffatomen bevorzugt sind.

Ebenso können Oligoribonukleotide verwendet werden, die α-Nukleoside enthalten.

Geeignete Basenmodifikationen werden z.B. in der US 6,187 578 und der WO 99/53101 beschrieben, auf die hiermit ausdrücklich Bezug genommen wird. Als vorteilhaft hat sich eine Modifikation eines oder mehrerer Pyrimidine in Position 5 mit I, Br, Cl, NH₃ und N₃ erwiesen.

Die Synthese modifizierter und nicht modifizierter Oligoribonukleotide sowie weitere geeignete Modifikationsmöglichkeiten sind in der Literatur beschrieben. Zudem wird die Herstellung modifizierter und nichtmodifizierter Oligoribonukleotide inzwischen auch von zahlreichen Firmen als Dienstleitung angeboten, beispielsweise von den Firmen Dharmacon, 1376 Miners Drive#101, Lafayette, CO 80026, USA, Xeragon Inc., Genset Oligos und Ambion. Die Herstellung von Oligoribonukleotiden wird darüber hinaus auch in der US 5,986,084 beschrieben.

Zur Erhöhung der Stabilität und/oder der Penetration können die Oligoribonukleotide auch in verkapselter Form verwendet werden, beispielsweise verkapselt in Liposomen. Außerdem können sie durch die Zugabe von Cyclodextrinen stabilisiert werden.

Cyclodextrine werden auch als Cycloamylosen und Cycloglucane bezeichnet. Es handelt sich bei den Cyclodextrinen um zyklische Oligosaccharide bestehend aus α-1,4 verknüpften Glucosebausteinen. In der Regel sind sechs bis acht Glucosebausteine (α-, β-, bzw. γ-Cyclodextrin) miteinander verbunden. Cyclodextrine werden bei Einwirkung von *Bacillus macerans* auf Stärke erhalten. Sie besitzen einen hydrophoben Innenraum und eine hydrophile Außenseite. Erfindungsgemäss sind sowohl die Cyclodextrine selbst, insbesondere α-Cyclodextrin, β-Cyclodextrin und γ-Cyclodextrin, als auch Derivate davon geeignet.

Erfindungsgemäss werden das oder die Cyclodextrine in kosmetischen und dermatologischen Zusammensetzungen vorzugsweise in einer Konzentration von 0.0005 bis 20.0 Gew.-%, insbesondere 0,01 bis 10 Gew.- % und besonders bevorzugt in einer Konzentration von 0.1 bis 5.0 Gew.-% eingesetzt.

Es ist erfindungsgemäss vorteilhaft native, polar- und/oder unpolar- substituierte Cyclodextrine einzusetzen. Hierzu gehören vorzugsweise aber nicht ausschließlich Methyl-, insbesondere random-Methyl-β-Cyclodextrin, Ethyl- sowie Hydroxypropyl-Cyclodextrine, beispielsweise Hydroxypropyl-β-Cyclodextrin und Hydroxypropyl-γ-Cyclodextrin. Die erfindungsgemäss besonders bevorzugten Cyclodextrinspezies sind γ-Cyclodextrin und Hydroxypropyl-β-Cylcodextrin.

Liposomen lassen sich auf an sich bekannte Weise unter Verwendung natürlicher Phospholipide, wie z.B. Phosphatidylcholin aus Eiern, Sojabohnen etc., oder synthetischer Phospholipide herstellen (vgl. G. Betageri (Herausgeber), "Liposome Drug Delivery Systems", Lancaster Techonomic Publishing Company 1993; Gregoriadis (Herausgeber), "Liposome Technology", CRC Press). Bevorzugte Verfahren und Materialien zur Herstellung von Liposomen werden in der WO 99/24018 beschrieben.

Doppelsträngige Oligoribonukleotide können zudem modifiziert werden, um einer Dissoziation in die Einzelstränge entgegenzuwirken, beispielsweise durch eine oder mehrere kovalente, koordinative oder ionische Bindungen. Oligoribonukleotide ohne derartige Modifikationen sind jedoch bevorzugt.

Die Nukleotide in den RNA Molekülen können weiterhin auch "non-standard" Nukleotide, wie z.B. nicht natürlich vorkommende Nukleotide oder Desoxyribonukleotide umfassen.

Erfindungsgemäss sind solche Oligoribonukleotide bevorzugt, die die Expression des jeweiligen Zielgens im Vergleich zu unbehandelten Zellen um mindestens 30 %, vorzugsweise um mindestens 50 %, besonders bevorzugt um mindestens 80 % und ganz besonders bevorzugt um mindestens 85 % inhibieren. Falls erforderlich, wird zur Messung der Inhibierung die Expression des Zielgens in den Zellen zunächst auf geeignete Weise induziert. Zur Bestimmung der Wirksamkeit der erfindungsgemässen Oligoribonukleotide werden vorzugsweise tumorale Zellen der Linie HeLaS3 verwendet. Die Oligoribonukleotide werden in die Zellen eingegracht und anschließend, ggf. nach Induktion der Expression des Zielgens, die Expressionsrate des Zielgen in diesen Zellen gemessen und mit derjenigen verglichen, die in Zellen gefunden wird, die nicht mit dem jeweiligen Oligoribonukleotid transfiziert wurden. Die genauen Bedingungen zur Messung der Inhibition finden sich in Beispiel 1.

Die erfindungsgemässen Oligoribonukleotide und deren Salze eignen sich besonders als wirksamer Bestandteil von pharmazeutischen und kosmetischen Zusammensetzungen, insbesondere solchen zur topischen Anwendung.

Es hat sich überraschenderweise herausgestellt, dass die Oligoribonukleotide nach dem Aufbringen der Zusammensetzungen auf die Haut die Expression der Gene inhibieren, die für Entzündungsprozesse der Haut verantwortlich sind, und so die Bildung und Verteilung von Entzündungsmediatoren nebenwirkungsfrei verhindern und auf diese Weise eine wirksame Behandlung und Prophylaxe von Irritationen und Entzündungen der Haut ermöglichen, ohne die Nachteile des Standes der Technik zu zeigen. Es wird angenommen, dass diese Wirkung darauf zurückzuführen ist, dass die erfindungsgemässen Oligoribonukleotide von den Zellen der Haut aufgenommen werden und intrazellulär den Abbau der mRNAs der genannten Gene durch RNAi induzieren, wobei Einzelheiten des Mechanismus dieser Reaktionskaskade noch nicht bekannt sind. Die Oligoribonukleotide eignen sich daher besonders zur Initüerung des Abbaus von mRNA von an der Entzündung der Haut beteiligten Strukturen und zur Inhibierung derartiger Strukturen in der Haut und insbesondere in Hautzellen.

Die erfindungsgemässen pharmazeutischen oder kosmetischen Zusammensetzungen enthalten vorzugsweise 0,00001 bis 10 Gew.%, besonders bevorzugt 0,0003 bis 3 Gew.-% und ganz besonders bevorzugt 0,01 bis 1,0 des oder der erfindungsgemässen Oligoribonukleotide, bezogen auf das Gesamtgewicht der Zusammensetzung. Bei der Verwendung von Oligorbinonukleotiden, die in Vektoren integriert sind, bezieht sich die obige Mengenangabe auf die Masse der in den Vektor integrierten Oligoribonukleotide, die Masse des Vektors selbst wird nicht berücksichtigt.

Erfindungsgemäss sind solche Zusammensetzungen bevorzugt, die ausschließlich solche Oligoribonukleotide enthalten, die die Expression eines oder mehrerer der oben genannten Gene, d.h. der Gene von an der Entzündung beteiligten Strukturen und insbesondere der genannten bevorzugten Gene inhibieren. Die erfindungsgemässen Zusammensetzungen können ein oder vorzugsweise mehrere Oligoribonukleotide enthalten. Hierbei kann es sich um Oligoribonukleotide handeln, die die Expression mehrerer unterschiedlicher an der Entzündung/ Irritation der Haut beteiligten Strukturen inhibieren, es können aber auch Gemische von Oligoribonukleotiden eingesetzt werden, die verschiedene Sequenzbereiche ein und desselben Gens oder derselben mRNA einer an der Hautentzündung beteiligten Struktur zum Ziel haben. Bevorzugt sind Zusammensetzungen, die 1 bis 5 und insbesondere 1 bis 3 verschiedene Oligoribonukleotide enthalten. Mischungen von Oligoribonukleotiden, die neben den genannten an der Hautentzündung beteiligten Strukturen unspezifisch die Aktivität einer Vielzahl von anderen Hautproteinen inhibieren oder induzieren sind unerwünscht, da praktisch keine Kontrolle von Nebenwirkungen möglich ist. Unter Hautproteinen werden solche Proteine verstanden, die in der Haut exprimiert werden. Ganz besonders bevorzugt sind Zusammensetzungen, die ein oder mehrere Oligoribonukleotide enthalten, die die Expression der Cyclooxygenase oder. der Lipoxygenase inhibieren.

Besonders bevorzugt sind weiterhin Zusammensetzungen, die jeweils mindestens ein Oligoribonukleotid enthalten, das gegen COX-2 gerichtet ist.

Ferner sind besonders bevorzugt Zusammensetzungen, die jeweils mindestens ein Oligoribonukleotid enthalten, das gegen LOX-5 und / oder FLAP gerichtet ist.

Außerdem sind besonders bevorzugt Zusammensetzungen, die jeweils mindestens ein Oligoribonukleotid enthalten, das gegen IL-1 α und/oder β und/oder IL-6 und/oder IL-8 und/oder TNF-α gerichtet ist.

Die Oligoribonukleotide und Zusammensetzungen eignen sich zur Behandlung und Prophylaxe unerwünschter Enzündungsreaktionen der Haut, insbesondere der oben beschriebenen Symptome. Sie eignen sich zur kosmetischen und therapeutischen Behandlung von unerwünschten Hautirritationen, die durch endogene und exogene Faktoren, insbesondere UV-Strahlung oder Rasur hervorgerufen werden. Die erfindungsgemässen Zusammensetzungen können Hautirritationen vorbeugen und vorhandene Irritationen dauerhaft und ohne das Risiko von Nebenwirkungen lindern. Zur Bestimmung der Wirksamkeit der erfindungsgemässen Oligoribonukleotide kann beispielsweise das in der WO02/053773 beschriebene Verfahren verwendet werden.

Die erfindungsgemässen Oligoribonukleotide eignen sich besonders zur Vorbeugung und Behandlung von Entzündungen der Haut, wie sie durch Sonnenbrand, aber auch durch Rasur hervorgerufen werden. In einer ebenso bevorzugten Form eignen sich die erfindungsgemässen Oligoribonukleotide zur Vorbeugung und Behandlung von Irritativen Hautzuständen wie sie durch exogenen Stress (z. B. UV-Strahlung, mechanische Belastung, Chemikalien) hervorgerufen werden. Ebenso eignet sich die erfindungsgemässen Oligoribonukleotide zur Beruhigung empfindlicher, zu Irritationen neigender Hauttypen.

Auf Grund ihrer prophylaktischen Wirkung eignen sich die erfindungsgemässen Oligoribonukleotide und Zusammensetzungen auch hervorragend zur Hautpflege.

Weiter eignen sich die erfindungsgemässen Zusammensetzungen zur Behandlung der durch UV-Strahlen, z.B. den ultravioletten Teil der Sonnenstrahlung, hervorgerufenen Hautschäden. UVB-Strahlen (290 bis 320 nm) verursachen beispielsweise Erytheme, Sonnenbrand oder sogar mehr oder weniger starke Verbrennungen. UVA-Strahlen (320 nm bis 400 nm) können Irritationen bei lichtempfindlicher Haut hervorrufen und führen zu einer Schädigung der elastischen und kollagenen Fasern des Bindegewebes, was die Haut vorzeitig altern läßt. Zudem sind sie Ursache zahlreicher phototoxischer und photoallergischer Reaktionen. Die erfindungsgemässen Oligoribonukleotide eignen sich auch zur Behandlung von z.B. durch UV-Strahlen hervorgerufenen Strukturschäden und Funktionsstörungen in der Epidermis und Dermis der Haut, wie beispielsweise von sichtbaren Gefäßerweiterungen, wie Teleangiektasien und Cuperosis, Hautschlaffheit und Ausbildung von Falten, lokalen Hyper-, Hypo- und Fehlpigmentierungen, wie z. B. Altersflecken, und vergrößerter Anfälligkeit gegenüber mechanischem Stress, wie z.B. Rissigkeit der Haut.

Weitere Anwendungsgebiete für die erfindungsgemässen Zusammensetzungen sind die Behandlung und Verhinderung der Alters- und/oder UV-induzierten Kollagendegeneration sowie dem Abbau von Elastin und Glykosaminoglykanen; von degenerative Erscheinungen der Haut, wie Elastizitätsverlust sowie Schwund der epidermalen und dermalen Zellschichten, der Bestandteile des Bindegewebes, der Retezapfen und Kapillargefäße) und/oder der Hautanhanggebilde; von umweltbedingten, z.B. durch ultraviolette Strahlung, Rauchen, Smog, reaktive Sauerstoffspezies, freie Radikale und dergleichen verursachte, negativen Veränderungen der Haut und der Hautanhanggebilde; von defizitären, sensitiven oder hypoaktiven Hautzuständen oder defizitären, sensitiven oder hypoaktiven Zustände von Hautanhanggebilden; der Verringerung der Hautdicke; von Hauterschlaffung und/oder Hautermüdung; von Veränderungen des transepidermalen Wasserverlustes und des normalen Feuchtigkeitsgehaltes der Haut; von Veränderung des Energiestoffwechsels der gesunden Haut; von Abweichungen von der normalen Zell-Zell-Kommunikation in der Haut, die sich z.B. durch Faltenbildung äußern kann; von Veränderungen der normalen Fibroblasten- und Keratinozytenproliferation; von Veränderungen der normalen Fibroblasten- und Keratinozytendifferenzierung; von polymorpher Lichtdermatose, Vitiligo; von Wundheilungsstörungen; von Störungen der normalen Kollagen-, Hyaluronsäure-, Elastin- und Glykosaminoglykan-Homeostase; der gesteigerten Aktivierung proteolytischer Enzyme in der Haut, wie z. B. von Metalloproteinasen.

Erfindungsgemäss sind Zusammensetzungen zur topischen Anwendung bevorzugt. Die Zusammensetzungen können in allen galenische Formen vorliegen, die gewöhnlicherweise für eine topische Applikation eingesetzt werden, z.B. als Lösung, Creme, Salbe, Lotion, Shampoo, das heißt Emulsion vom Typ Wasser-in-Öl (W/O) oder vom Typ Öl-in-Wasser (O/W), multiple Emulsion, beispielsweise vom Typ Wasser-in-Öl-in-Wasser (W/O/W), oder Öl-in-Wasser-in-Öl (O/W/O), Hydrodispersion oder Lipodispersion, Pickering-Emulsion, Gel, fester Stift oder Aerosol.

Die kosmetische oder medizinische Behandlung der genannten Indikationen erfolgt in der Regel durch ein- oder mehrmaligen Auftrag der erfindungsgemässen Zusammensetzungen auf die Haut, vorzugsweise auf die betroffenen Hautstellen.

Der erfindungsgemässen Zusammensetzungen eignen sich zur kosmetischen und therapeutischen, d.h. insbesondere dermatologischen Anwendung.
Unter kosmetischer Hautpflege ist in erster Linie zu verstehen, dass die natürliche Funktion der Haut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen.

Zur kosmetischen Anwendung enthalten die erfindungsgemässen Zusammensetzungen daher vorzugsweise solche Komponenten, die für die genannten Zwecke geeignet sind. Solche Substanzen sind dem Fachmann an sich bekannt. Beispielsweise können ein oder mehrere Antisense Oligoribonukleotide in übliche kosmetische und dermatologische Zubereitungen eingearbeitet werden, welche in verschiedenen Formen vorliegen können.

Gemäss einer besonders bevorzugten Ausführungsform liegen die erfindungsgemässen Zusammensetzungen zur kosmetischen Anwendung als Emulsion vor, z.B. in Form einer Creme, einer Lotion, einer kosmetischen Milch. Diese enthalten neben den genannten Oligoribonukleotiden weitere Komponenten wie z.B. Fette, Öle, Wachse und/oder andere Fettkörper, sowie Wasser und einen oder mehrere Emulgatoren, wie sie üblicherweise für einen solchen Typ der Formulierung verwendet werden.

Emulsionen enthalten in der Regel eine Lipid- oder Ölphase eine wäßrige Phase und vorzugsweise auch einen oder mehrere Emulgatoren. Besonders bevorzugt sind Zusammensetzungen, die darüber hinaus auch ein oder mehrere Hydrocolloide enthalten.

Die erfindungsgemässen Zusammensetzungen enthalten vorzugsweise 0,001 bis 35 Gew.-%, besonders bevorzugt 2 bis 15 Gew.-% Emulgator, 0,001 bis 45 Gew.-%, besonders bevorzugt 10 bis 25 Gew.-% Lipid und 10 bis 95 Gew.-%, besonders bevorzugt 60 bis 90 Gew.-% Wasser.

Die Lipidphase der erfindungsgemässen kosmetischen oder dermatologischen Emulsionen kann vorteilhaft gewählt werden aus folgender Substanzgruppe: (1) Mineralöle, Mineralwachse; (2) Öle, wie Triglyceride der Caprin- oder der Caprylsäure, ferner natürliche Öle wie z.B. Rizinusöl; (3) Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren; (4) Alkylbenzoate; (5) Silikonöle wie Dimethylpolysiloxane, Diethylpolysiloxane, Diphenylpoly-siloxane sowie Mischformen daraus.

Wenn nicht anders angegeben werden hierin unter niedriger C-Zahl vorzugsweise 1 bis 5, besonders bevorzugt 1 bis 3 und ganz besonders bevorzugt 3 Kohlenstoffatome verstanden.

Die Ölphase der Emulsionen der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n -Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren ei ner Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden. Solche Silicone oder Silikonöle können als Monomere vorliegen, welche in der Regel durch Strukturelemente charakterisiert sind, wie folgt:

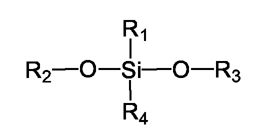

Als erfindungsgemäss vorteilhaft einzusetzenden linearen Silicone mit mehreren Siloxyleinheiten werden im allgemeinen durch Strukturelemente charakterisiert wie folgt: 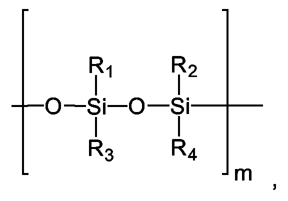 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). m kann dabei Werte von 2 - 200.000 annehmen. Aryl steht hierin, wenn nicht anders angegeben, vorzugsweise für Phenyl.

Erfindungsgemäss vorteilhaft einzusetzende cyclische Silicone werden im allgemeinen durch Strukturelemente charakterisiert, wie folgt 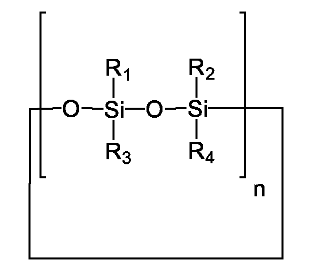 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). n kann dabei Werte von 3/2 bis 20 annehmen. Gebrochene Werte für n berücksichtigen, dass ungeradzahlige Anzahlen von Siloxylgruppen im Cyclus vorhanden sein können.

Vorteilhaft wird Cyclomethicon (z.B. Decamethylcyclopentasiloxan) als erfindungsgemäss zu verwendendes Silikonöl eingesetzt. Aber auch andere Silikonöle sind vorteilhaft im Sinne der vorliegenden Erfindung zu verwenden, beispielsweise Undecamethylcyclotrisiloxan, Polydimethylsiloxan, Poly(methylphenylsiloxan), Cetyldimethicon, Behenoxydimethicon.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxan-polyalkyl-polyether-copolymere wie das Cetyl-Dimethicon-Copolyol, das (Cetyl-Dimethicon-Copolyol (und) Polyglyceryl-4-Isostearat (und) Hexyllaurat).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemässen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate.

Erfindungsgemässe als Emulsionen vorliegenden Zubereitungen enthalten vorzugsweise einen oder mehrere Emulgatoren. Diese Emulgatoren können vorteilhaft gewählt werden aus der Gruppe der nichtionischen, anionischen, kationischen oder amphoteren Emulgatoren.

Unter den nichtionischen Emulgatoren befinden sich (1) Partialfettsäureester und Fettsäureester mehrwertiger Alkohole und deren ethoxylierte Derivate (z. B. Glycerylmonostearate, Sorbitanstearate, Glycerylstearylcitrate, Sucrosestearate); (2) ethoxylierte Fettalkohole und Fettsäuren; (3) ethoxilierte Fettamine, Fettsäureamide, Fettsäurealkanolamide; (4) Alkylphenolpolyglycolether (z.B. Triton X).

Unter den anionischen Emulgatoren befinden sich Seifen (z. B. Natriumstearat); Fettalkoholsulfate; Mono-, Di- und Trialkylphosphosäureester und deren Ethoxylate.
Unter den kationischen Emulgatoren befinden sich quaternäre Ammoniumverbindungen mit einem langkettigen aliphatischen Rest z.B. Distearyldimonium Chloride.

Unter den amphoteren Emulgatoren befinden sich Alkylamininoalkancarbonsäuren, Betaine, Sulfobetaine, Imidazolinderivate.

Weiterhin gibt es natürlich vorkommende Emulgatoren, zu denen Bienenwachs, Wollwachs, Lecithin und Sterole gehören.

O/W-Emulgatoren können beispielsweise vorteilhaft gewählt werden aus der Gruppe der polyethoxylierten bzw. polypropoxylierten bzw. polyethoxylierten und polypropoxylierten Produkte, z.B. der Fettalkoholethoxylate, der ethoxylierten Wollwachsalkohole, der Polyethylenglycolether der allgemeinen Formel R-O-(-CH₂-CH₂-O-)ₙ-R', der Fettsäureethoxylate der allgemeinen Formel R-COO-(-CH₂-CH₂-O-)ₙ-H, der veretherten Fettsäureethoxylate der allgemeinen Formel R-COO-(-CH₂-CH₂-O)ₙ-R', der veresterten Fettsäureethoxylate der allgemeinen Formel R-COO-(-CH₂-CH₂-O-)ₙ C(O)-R', der Polyethylenglycolglycerinfettsäureester, der ethoxylierten Sorbitanester, der Cholesterinethoxylate, der ethoxylierten Triglyceride, der Alkylethercarbonsäuren der allgemeinen Formel R-O-(-CH₂-CH₂-O-)ₙ-CH₂-COOH, der Polyoxyethylensorbitolfettsäureester, der Alkylethersulfate der allgemeinen Formel R-O-(-CH₂-CH₂-O-)ₙ-SO₃-H, der Fettalkoholpropoxylate der allgemeinen Formel R-O-(-CH₂-CH(CH₃)-O-)ₙ-H, der Polypropylenglycolether der allgemeinen Formel R-O-(-CH₂-CH(CH₃)-O-)ₙ-R', der propoxylierten Wollwachsalkohole, der veretherten Fettsäurepropoxylate, R-COO-(-CH₂-CH(CH₃)-O-)ₙ-R', der veresterten Fettsäurepropoxylate der allgemeinen Formel R-COO-(-CH₂-CH(CH₃)-O-)ₙ-C(O)-R', der Fettsäurepropoxylate der allgemeinen Formel R-COO-(-CH₂-CH(CH₃)-O-)ₙ-H, der Polypropylenglycolglycerinfettsäureester, der propoxylierten Sorbitanester, der Cholesterinpropoxylate, der propoxylierten Triglyceride, der Alkylethercarbonsäuren der allgemeinen Formel R-O-(-CH₂-CH(CH₃)O-)ₙ-CH₂-COOH, der Alkylethersulfate bzw. die diesen Sulfaten zugrundeliegenden Säuren der allgemeinen Formel R-O-(-CH₂-CH(CH₃)-O-)ₙ-SO₃-H, der Fettalkoholethoxylate/propoxylate der allgemeinen Formel R-O-Xn-Yₘ-H, der Polypropylenglycolether der allgemeinen Formel R-O-Xn-Yₘ-R', der veretherten Fettsäurepropoxylate der allgemeinen Formel R-COO-Xₙ-Yₘ-R', der Fettsäureethoxylate/propoxylate der allgemeinen Formel R-COO-Xₙ-Yₘ-H.

Die Variablen n und m stehen in allen Fällen unabhängig voneinander jeweils für eine ganze Zahl von 1 bis 40, vorzugsweise 5 bis 30.

Erfindungsgemäss besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether (Steareth-20),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19), Polyethylenglycol(20)isostearylether (Isosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (Isolaureth-12).
Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(1 6)cetylstearylether (Ceteareth-16), Polyethylenglycol(1 7)cetylstearylether (Ceteareth-17), Polyethylenglycol(1 8)cetylstearylether (Ceteareth-18), Polyethylenglycol-(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,
Polyethylenglycol(12)isostearat, Polyethylenglycol(13)isostearat, Polyethylenglycol(14)-isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol-(22)isostearat, Polyethylenglycol(23)isostearat, Polyethylenglycol(24)isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat, Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol-(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol-(22)glyceryllaurat, Polyethylenglycol(23)glyceryllaurat, Polyethylenglycol(6)glycerylcaprat/caprinat, Polyethylenglycol(20)glyceryloleat, Polyethylenglycol-(20)glycerylisostearat, Polyethylenglycol(18)glyceryloleat/cocoat zu wählen.

Es ist ebenfalls günstig, die Sorbitanester aus der Gruppe Polyethylenglycol-(20)sorbitanmonolaurat, Polyethylenglycol(20)sorbitanmonostearat, Polyethylenglycol-(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemässe als Emulsionen vorliegenden Zubereitungen enthalten darüber hinaus vorzugsweise auch ein oder mehrere Hydrocolloide. Diese Hydrocolloide können vorteilhaft gewählt werden aus der Gruppe der Gummen, Polysaccharide, Cellulosederivate, Schichtsilikate, Polyacrylate und/oder anderen Polymeren.

Erfindungsgemässe als Hydrogele vorliegenden Zubereitungen enthalten ein oder mehrere Hydrocolloide. Diese Hydrocolloide können vorteilhaft aus der vorgenannten Gruppe gewählt werden.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können vorteilhaft auch Kieselsäuregele verwendet werden.

Unter den Polyacrylaten befinden sich z.B. Carbopol Typen der Firma Goodrich (Carbopol 980, 981, 1382, 5984, 2984, EDT 2001 oder Pemulen TR2).

Unter den Polymeren befinden sich z.B. Polyacrylamide (Seppigel 305), Polyvinylalkohole, PVP, PVP / VA Copolymere, Polyglycole.

Gemäss einer weiteren bevorzugten Ausführungsform werden die erfindungsgemäss verwendeten Oligoribonukleotide in wäßrige Systeme bzw. Tensidzubereitungen zur Reinigung der Haut und der Haare eingefügt.

Die erfindungsgemässen kosmetischen Zubereitungen enthalten neben den genannten Komponenten vorzugsweise auch Hilfsstoffe, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Verdickungsmittel, weichmachende Substanzen, anfeuchtende und/oder feuchthaltende Substanzen (Moisturizer), oder andere übliche Bestandteile einer kosmetischen Formulierung wie Polyole, Polymere, Schaumstabilisatoren, Elektrolyte, organische Lösungsmittel oder Silikonderivate, Antioxidantien und insbesondere UV-Absorber.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen. Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Glycerin wird bei der Verwendung als Moisturizer vorzugsweise in einer Menge von 0,05-30 Gew.%, besonders bevorzugt sind 1-10%, eingesetzt.

Die kosmetischen Zusammensetzungen können vorteilhaft auch einen oder mehrere der folgenden natürlichen Wirkstoffe oder ein Derivat davon enthalten: alpha-Liponsäure, Phytoen, D-Biotin, Coenzym Q10, alpha Glucosylrutin, Carnitin, Carnosin, natürliche und/oder synthetische Isoflavonoide, Kreatin, Hopfen- bzw. Hopfen-Malz-Extrakt, Taurin. So zeigte sich, dass Wirkstoffe zur positiven Beeinflussung der Altershaut, die die Entstehung von Falten oder auch bestehenden Falten vermindern, wie Biochinone und insbesondere Ubichinon Q10, Soja, Creatinin, Creatin, Liponamid, oder die Restrukturierung des Bindegewebes fördern, wie Isoflavon, in den erfindungsgemässen Formulierungen sehr gut verwendet werden können. Auch zeigte sich, dass sich die Formulierungen in besonderer Weise zur Kombination mit Wirkstoffen zur Unterstützung der Hautfunktionen bei trockener Haut, insbesondere alterstrockener Haut, wie Serinol und Osmolyte, z.B. Taurin, eignen. Auch erwies sich die Einarbeitung von Modulatoren der Pigmentierung als vorteilhaft. Hier sind Wirkstoffe zu nennen, die die Pigmentierung der Haut vermindern und so zu einer kosmetisch gewünschten Aufhellung der Haut führen und/oder das Auftreten von Altersflecken reduzieren und/oder bestehende Altersflecken aufhellen (Tyrosinsulfat, Dioic acid (8-Hexadecen-1,16-dicarbonsäure), Liponsäure und Liponamid, verschiedene Extrakte des Süßholzes, Kojisäure, Hydrochinon, Arbutin, Fruchtsäuren, insbesondere Alpha-Hydroxy-Säuren (AHAs), Bearberry (Uvae ursi), Ursolsäure, Ascorbinsäure, Grüntee-Extrakte).

Gemäss einer besonders bevorzugten Ausführungsform enthalten die erfindungsgemässen Zusammensetzungen einen oder mehrere UV-Absorber. Bevorzugte UV-Absorber sind solche, die im Bereich der UVB- und/oder UVA-Strahlen absorbieren.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt. Bevorzugt sind Filter mit einem Absorptionsmaximum im Bereich von 308 nm, da hier das Maximum der Erythemwirksamkeit des Sonnenlichtes liegt.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Die Zubereitungen gemäss der Erfindung enthalten vorteilhaft Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Weitere vorteilhafte UV-A-Filtersubstanzen sind die Phenylen-1,4-bis-(2-benzimidazyl)-3,3'-5,5'-tetrasulfonsäure 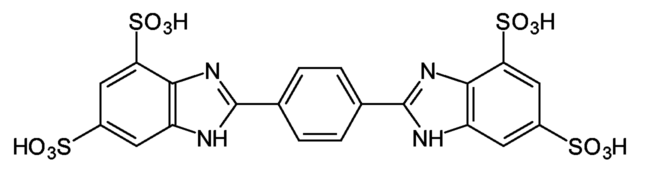 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz 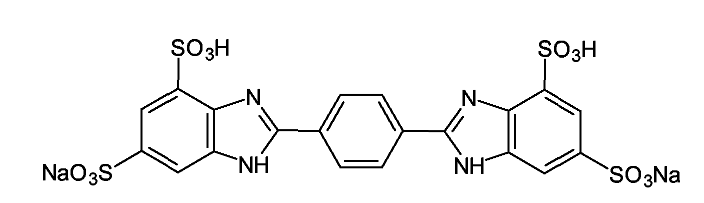 mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

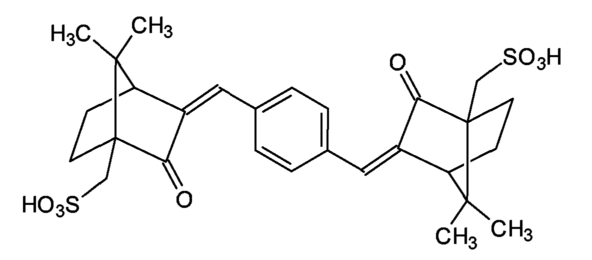

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 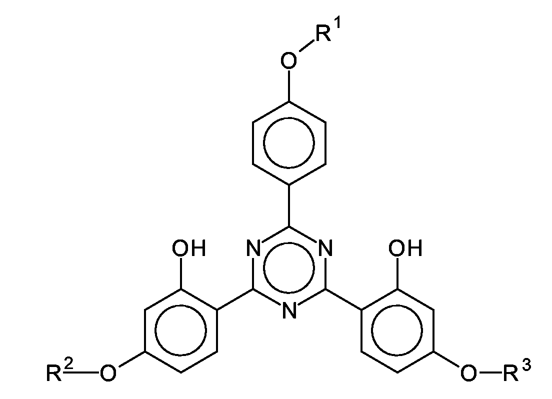 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCl: Aniso Triazin), welches unter der Handelsbezeichnung Tinosorb® S bei der CIBA-Chemikalien GmbH erhältlich ist, und das 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin (INCl: Octyl Triazone), welches von der BASF Aktiengesellschaft unter der Warenbezeichnung UVINUL® T 150 vertrieben wird.

Auch andere UV-Filtersubstanzen, welche das Strukturmotiv 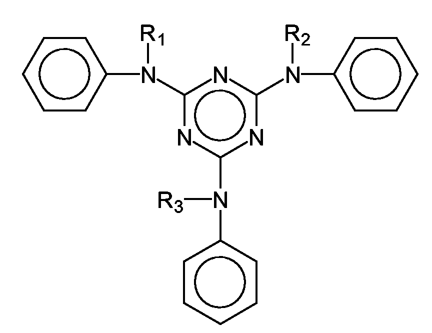 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 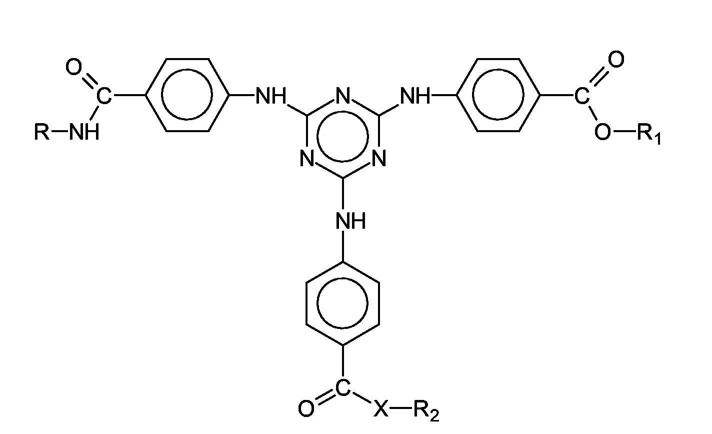 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Al kylgruppen, oder ein Wasserstoffatom, ein Alkalimetallatom, eine Ammoniumgruppe oder eine Gruppe der Formel 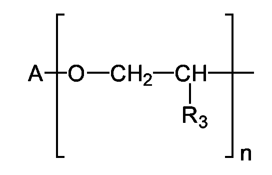 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 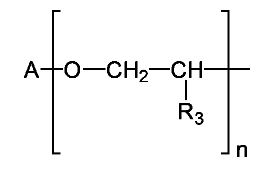 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
wenn X ein Sauerstoffatom darstellt.

Eine besonders vorteilhafte UV-Filtersubstanz im Sinne der vorliegenden Erfindung ist ferner ein unsymmetrisch substituiertes s-Triazin, dessen chemische Struktur durch die Formel 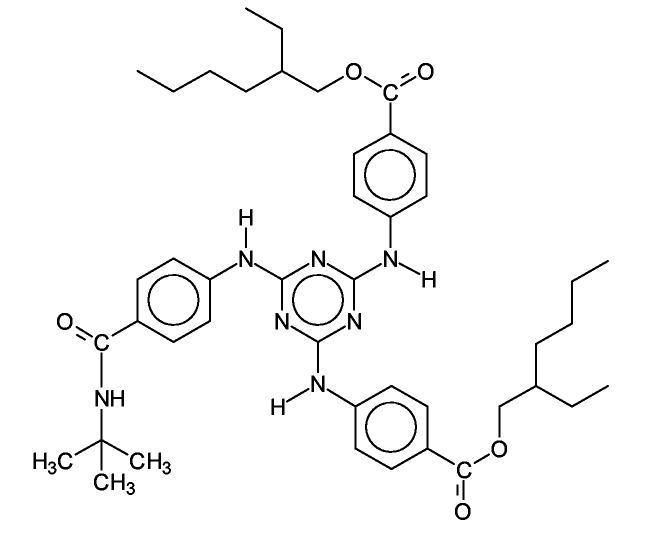 wiedergegeben wird, welches im Folgenden auch als Dioctylbutylamidotriazon (INCI: Dioctylbutamidotriazone) bezeichnet wird und unter der Handelsbezeichnung UVA-SORB HEB bei Sigma 3V erhältlich ist.

Auch in der Europäischen Offenlegungsschrift EP 775 698 werden vorteilhaft einzusetzende Bis-Resorcinyltriazinderivate beschrieben, deren chemische Struktur durch die generische Formel 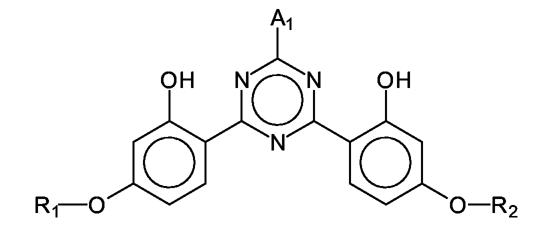 wiedergegeben wird, wobei R₁ , R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxypropyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-([4-(1',1',1',3',5',5',5'-Heptamethylsiloxy-2"-methylpropyloxy)-2-hydroxy]-phenyl-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel 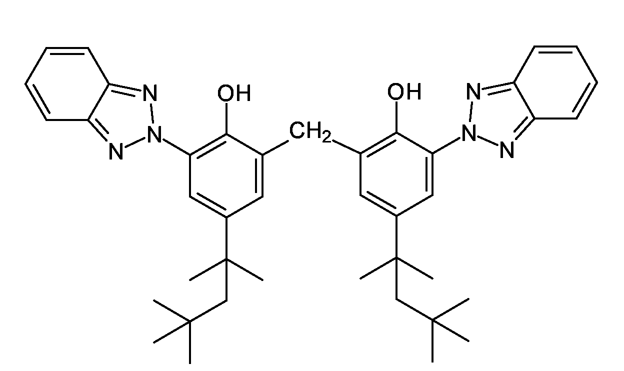 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(tri methylsilyl)oxy]d i-siloxanyl]propyl]-phenol (CAS-Nr.: 155633-54-8) mit der INCI-Bezeichnung Drometrizole Trisiloxane, welches durch die chemische Strukturformel 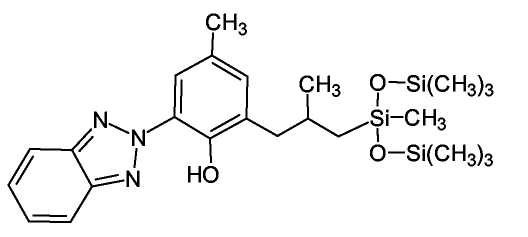 gekennzeichnet ist.

Die UV-B-Filter können öllöslich oder wasserlöslich sein. Vorteilhafte öllösliche UV-B-Filtersubstanzen sind z. B.: 3-Benzylidencampher-Derivate, vorzugsweise 3-(4-Methylbenzyliden)campher, 3-Benzylidencampher; 4-Aminobenzoesäure-Derivate, vorzugsweise 4-(Dimethylamino)-benzoesäure(2-ethylhexyl)ester, 4-(Dimethylamino)benzoesäureamylester; 2,4,6-Trianilino-(p-carbo-2'-ethyl-1'-hexyloxy)-1,3,5-triazin; Ester der Benzalmalonsäure, vorzugsweise 4-Methoxybenzalmalonsäuredi(2-ethylhexyl)ester; Ester der Zimtsäure, vorzugsweise 4-Methoxyzimtsäure(2-ethylhexyl)ester, 4-Methoxyzimtsäureisopentylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie an Polymere gebundene UV-Filter.

Vorteilhafte wasserlösliche UV-B-Filtersubstanzen sind z. B. Salze der 2-Phenylbenzimidazol-5-sulfonsäure, wie ihr Natrium-, Kalium- oder ihr Triethanolammonium-Salz, sowie die Sulfonsäure selbst; Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäss vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

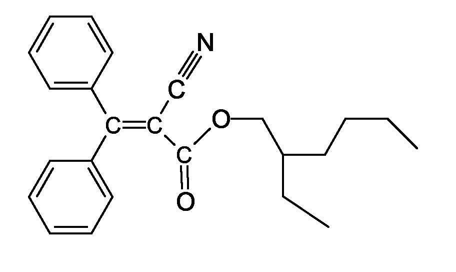

Es kann auch von erheblichem Vorteil sein, polymergebundene oder polymere UV-Filtersubstanzen in Zubereitungen gemäss der vorliegenden Erfindung zu verwenden, insbesondere solche, wie sie in der WO-A-92/20690 beschrieben werden.

Ferner kann es gegebenenfalls von Vorteil sein, erfindungsgemäss weitere UV-A-und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Weiterhin können die erfindungsgemässen Zusammensetzungen Antioxidantien zum Schutz der kosmetischen Zubereitung selbst bzw. zum Schutz der Bestandteile der kosmetischen Zubereitungen vor schädlichen Oxidationsprozessen enthalten.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoe harzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäss geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.
Erfindungsgemässe kosmetische und Therapeutische Zubereitungen enthalten vorteilhaft außerdem anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden. Besonders bevorzugt handelt es sich um Pigmente auf der Basis von TiO₂.

Es ist besonders vorteilhaft im Sinne der vorliegenden Erfindung, wenngleich nicht zwingend, wenn die anorganischen Pigmente in hydrophober Form vorliegen, d.h., dass sie oberflächlich wasserabweisend behandelt sind. Diese Oberflächenbehandlung kann darin bestehen, dass die Pigmente nach an sich bekannten Verfahren mit einer dünnen hydrophoben Schicht versehen werden.

Eines solcher Verfahren besteht beispielsweise darin, dass die hydrophobe Oberflächenschicht nach einer Rektion gemäss

n TiO₂ + m (RO)₃ Si-R' -> n TiO₂ (oberfl.)

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte TiO₂-Pigmente sind beispielsweise unter den Handelsbezeichnungen MT 100 T von der Firma TAYCA, ferner M 160 von der Firma Kemira sowie T 805 von der Firma Degussa erhältlich.

Erfindungsgemässe Zubereitungen können, zumal wenn kristalline oder mikrokristalline Festkörper, beispielsweise anorganische Mikropigmente in die erfindungsgemässen Zubereitungen eingearbeitet werden sollen, auch anionische, nichtionische und/oder amphotere Tenside enthalten. Tenside sind amphiphile Stoffe, die organische, unpolare Substanzen in Wasser lösen können.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden, nämlich anionische Tenside, kationische Tenside, amphotere Tenside und nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | |
|---|---|
| pH=2 | RNH₂⁺CH₂CH₂COOH X⁻ (X⁻ = beliebiges Anion, z.B. Cl⁻) |
| pH=7 | RNH₂⁺CH₂CH₂COO⁻ |
| pH=12 | RNHCH₂CH₂COO⁻ B⁺ (B⁺ = beliebiges Kation, z.B. Na⁺) |

Typisch für nicht-ionische Tenside sind Polyether-Ketten. Nicht-ionische Tenside bilden in wäßrigem Medium keine Ionen.

Vorteilhaft zu verwendende anionische Tenside sind:
Acylaminosäuren (und deren Salze), wie (1) Acylglutamate, beispielsweise Natriumacylglutamat, Di-TEA-palmitoylaspartat und Natrium Caprylic/ Capric Glutamat; (2) Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen; (3) Sarcosinate, beispielsweise Myristoyl Sarcosin, TEA-lauroyl Sarcosinat, Natriumlauroylsarcosinat und Natriumcocoylsarkosinat; (4) Taurate, beispielsweise Natriumlauroyltaurat und Natriummethylcocoyltaurat; (5) Acyllactylate, wie Lauroyllactylat und Caproyllactylat; (6) Alaninate;
Carbonsäuren und Derivate, wie beispielsweise Laurinsäure, Aluminiumstearat, Magnesiumalkanolat und Zinkundecylenat; Ester-Carbonsäuren, beispielsweise Calciumstearoyllactylat, Laureth-6 Citrat und Natrium PEG-4 Lauramidcarboxylat; Ether-Carbonsäuren, beispielsweise Natriumlaureth-13 Carboxylat und Natrium PEG-6 Cocamide Carboxylat;
Carbonsäuren, Ester-Carbonsäuren und Ether-Carbonsäuren enthalten vorzugsweise 1 bis 50 und insbesondere 2 bis 30 Kohlenstoffatome.
Phosphorsäureester und Salze, wie beispielsweise DEA-Oleth-10-Phosphat und Dilaureth-4 Phosphat;
Sulfonsäuren und Salze, wie (1) Acylisethionate, z.B. Natrium-/ Ammoniumcocoylisethionat; (2) Alkylarylsulfonate; (3) Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat; (4) Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat;
Schwefelsäureester, wie (1) Alkylethersulfat, beispielsweise Natrium-, Ammonium-, Magnesium-, MIPA-, TIPA- Lau rethsulfat, Natriummyrethsulfat und Natrium C₁₂₋₁₃ Pa-rethsulfat; (2) Alkylsulfate, beispielsweise Natrium-, Ammonium- und TEA- Laurylsulfat. Vorteilhaft zu verwendende kationische Tenside sind Alkylamine, Alkylimidazole, Ethoxylierte Amine und Quaternäre Tenside sowie Esterquats.

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäss verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl-oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Vorteilhaft zu verwendende amphotere Tenside sind (1)Acyl-/dialkylethylendiamin, beispielsweise Natriumacylamphoac etat, Dinatriumacylamphodipropionat, Dinatriumalkylamphodiacetat, Natriumacylamphohydroxypropylsulfonat, Dinatriumacylamphodiacetat und Natriumacylamphopropionat; (2) N-Alkylaminosäuren, beispielsweise Aminopropylalkylglutamid, Alkylaminopropionsäure, Natriumalkylimidodipropionat und Lauroamphocarboxyglycinat.

Vorteilhaft zu verwendende nicht-ionische Tenside sind (1) Alkohole; (2) Alkanolamide, wie Cocamide MEA/ DEA/ MIPA; (3) Aminoxide, wie Cocoamidopropylaminoxid; (4) Ester, die durch Veresterung von Carbonsäuren mit Ethylenoxid, Glycerin, Sorbitan oder anderen Alkoholen entstehen; (5) Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid; (6) Sucroseester, -ether; (7) Polyglycerinester, Diglycerinester, Monoglycerinester; (8) Methylglucosester, Ester von Hydroxysäuren.

Vorteilhaft ist ferner die Verwendung einer Kombination von anionischen und/oder amphoteren Tensiden mit einem oder mehreren nicht-ionischen Tensiden.

Die oberflächenaktive Substanz kann in einer Konzentration zwischen 1 und 95 Gew.-% in den erfindungsgemässen Zubereitungen vorliegen, bezogen auf das Gesamtgewicht der Zubereitungen.

Zubereitungen zur medizinischen Anwendung unterscheiden sich in ihrer Zusammensetzung nicht von den kosmetischen Produkten und können ebenso die oben genannten Stoffe enthalten. Sie unterscheiden sich von diesen in erster Linie dadurch, dass sie ein spezielles Zulassungsverfahren durchlaufen müssen.

Im folgenden wird die Erfindung anhand von Ausführungsbeispielen näher erläutert. In den Beispielen beziehen sich alle Zahlenangaben auf Gew.-%, sofern nichts Anderes angegeben ist.

### Beispiele

### Beispiel 1: Herstellung von PIT Emulsionen

Durch Mischen der in der Tabelle angegebenen Komponenten wurden Phasen-Inversions-Temperatur-Emulsionen (PIT-Emulsionen) der ebenfalls angegebenen Zusammensetzung hergestellt. Als Oligoribonukleotid wurde dsRNA verwendet, die durch Hybridisieren der Sequenzen SEQ ID NOs 120 und 135 erhalten wurde. Die dsRNA weist 3'-ständig jeweils zwei überstehende dT-Reste auf. Die dsRNA ist für die cDNA der Cycloxygenase spezifisch und inhibiert die Expression des Gens dieses Enzyms durch RNA-Interferenz. Sie wird daher als anti-Cyclooxygenase dsRNA bezeichnet. Die übrigen in den Beispielen verwendeten Abkürzungen sind entsprechend zu verstehen.

**Tabelle 1:**

| **PIT - Emulsionen** | | | | | |
|---|---|---|---|---|---|
| **Emulsion Nr.** | **1** | **2** | **3** | **4** | **5** |
| Glycerinmonostearat selbstemulgierend | 0,50 | | 3,00 | 2,00 | 4,00 |
| Polyoxyethylen(12)cetylstearylether | | 5,00 | | 1,00 | 1,50 |
| Polyoxyethylen(20)cetylstearylether | | | | 2,00 | |
| Polyoxyethylen(30)cetylstearylether | 5,00 | | 1,00 | | |
| Stearylalkohol | | | 3,00 | | 0,50 |
| Cetylalkohol | 2,50 | 1,00 | | 1,50 | |
| 2-Ethylhexyl Methoxyzinnamat | | | | 5,00 | 8,00 |
| 2,4-Bis-(4-(2-ethyl-hexyloxy-)2-hydroxyl)-phenyl)-6-(4-methoxyphenyl)-(1,3,5)-triazin | | 1,50 | | 2,00 | 2,50 |
| 1-(4-tert-Butylphenyl)-3-(4-methoxyphenyl)-1,3-propandion | | | 2,00 | | |
| Diethylhexyl Butamidotriazon | 1,00 | 2,00 | | 2,00 | |
| Ethylhexyl Triazon | 4,00 | | 3,00 | 4,00 | |
| 4-Methylbenzyliden Campher | | 4,00 | | | 2,00 |
| Octocrylen | | 4,00 | | | 2,50 |
| Phenylen-1,4-bis-(mononatrium, 2-benzimidazyl-5,7-disulfonsaeure | | | 0,50 | | 1,50 |
| Phenylbenzimidazol Sulfonsäure | 0,50 | | | 3,00 | |
| C12-15 Alkyl Benzoat | | 2,50 | | | 5,00 |
| Titandioxid | 0,50 | 1,00 | | 3,00 | 2,00 |
| Zinkoxid | 2,00 | | 3,00 | 0,50 | 1,00 |
| Dicaprylylether | | | 3,50 | | |
| Butylenglycol-Dicaprylat/-Dicaprat | 5,00 | | | 6,00 | |
| Dicaprylylcarbonat | | | 6,00 | | 2,00 |
| Dimethicon Polydimethylsiloxan | | 0,50 | 1,00 | | |
| Phenylmethylpolysiloxan | 2,00 | | | 0,50 | 0,50 |
| Shea-Butter (Sheabutter) | | 2,00 | | | 0,50 |
| PVP Hexadecencopolymer | 0,50 | | | 0,50 | 1,00 |
| Glycerin | 3,00 | 7,50 | 5,00 | 7,50 | 2,50 |
| Tocopherolacetat | 0,50 | | 0,25 | | 1,00 |
| anti-Cyclooxygenase-dsRNA (dsRNA aus SEQ ID NOs 120 und 135) | 0,10 | 0,10 | | 0,10 | 0,10 |
| Konservierungsmittel | q.s. | q.s. | q.s. | q.s. | q.s. |
| Ethanol | 3,00 | 2,00 | 1,50 | | 1,00 |
| Parfum | q.s. | q.s. | q.s. | q.s. | q.s. |
| Wasser | ad. 100 | ad. 100 | ad. 100 | ad. 100 | ad. 100 |

Auf analoge Weise wurde eine PIT-Emulsion unter Verwendung von dsRNA hergestellt, die durch Hybridisieren der Sequenzen SEQ NOs 130 und 131 erhalten wurde. Die Mengenabgaben zu anti-COX-2-dsRNA beziehen sich auf die Gesamtmenge an dsRNA, die sich aus den genannten, jeweiligen Sequenzen (SEQ IDs) zusammensetzt.

### Beispiel 2: Herstellung von Cremes auf der Basis von ÖI-in-Wasser-Emulsionen

Durch Mischen der in der Tabelle angegebenen Komponenten wurden Cremes der ebenfalls angegebenen Zusammensetzung hergestellt.

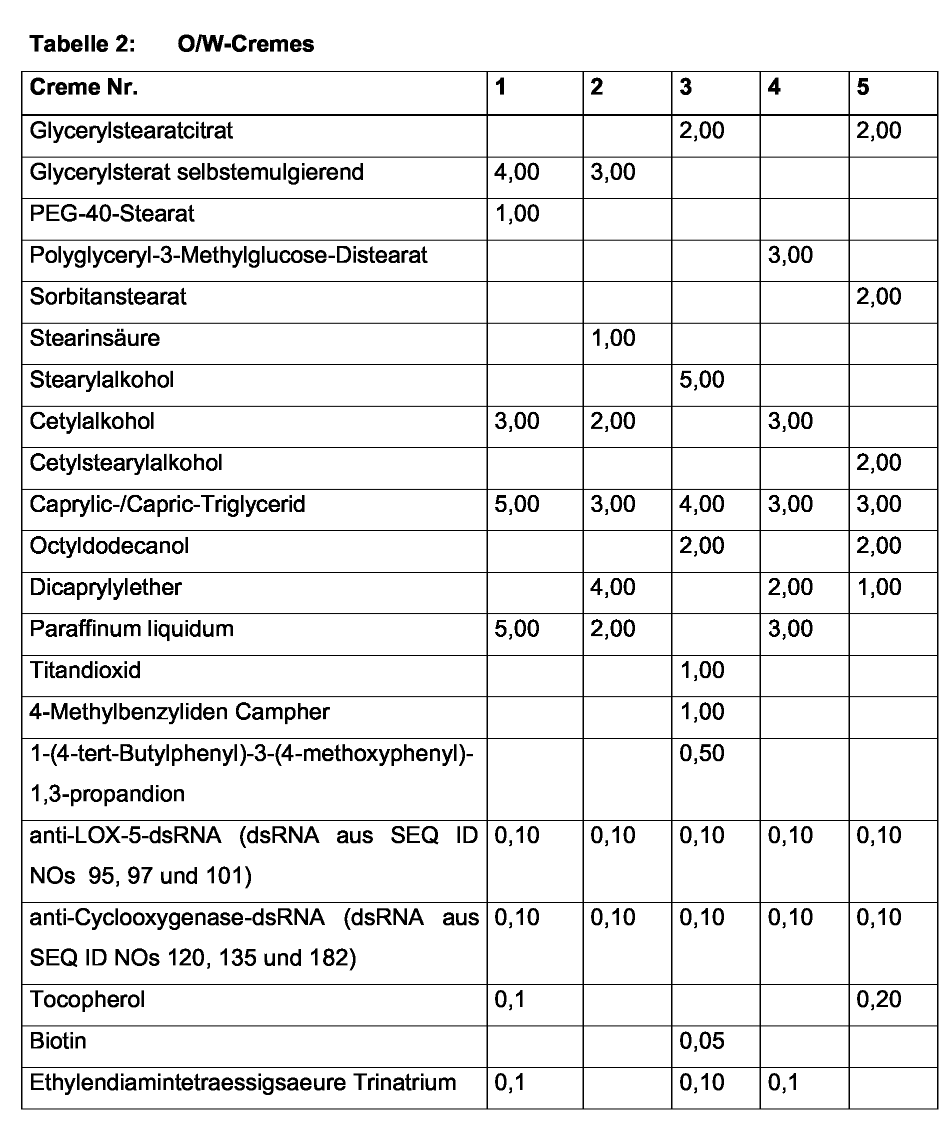

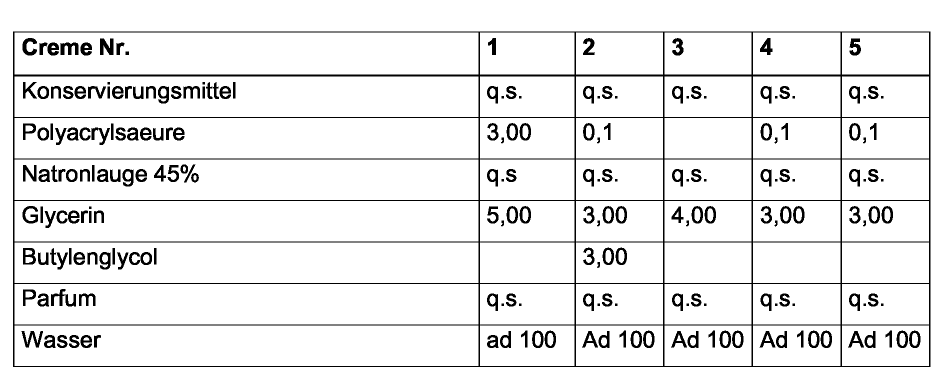

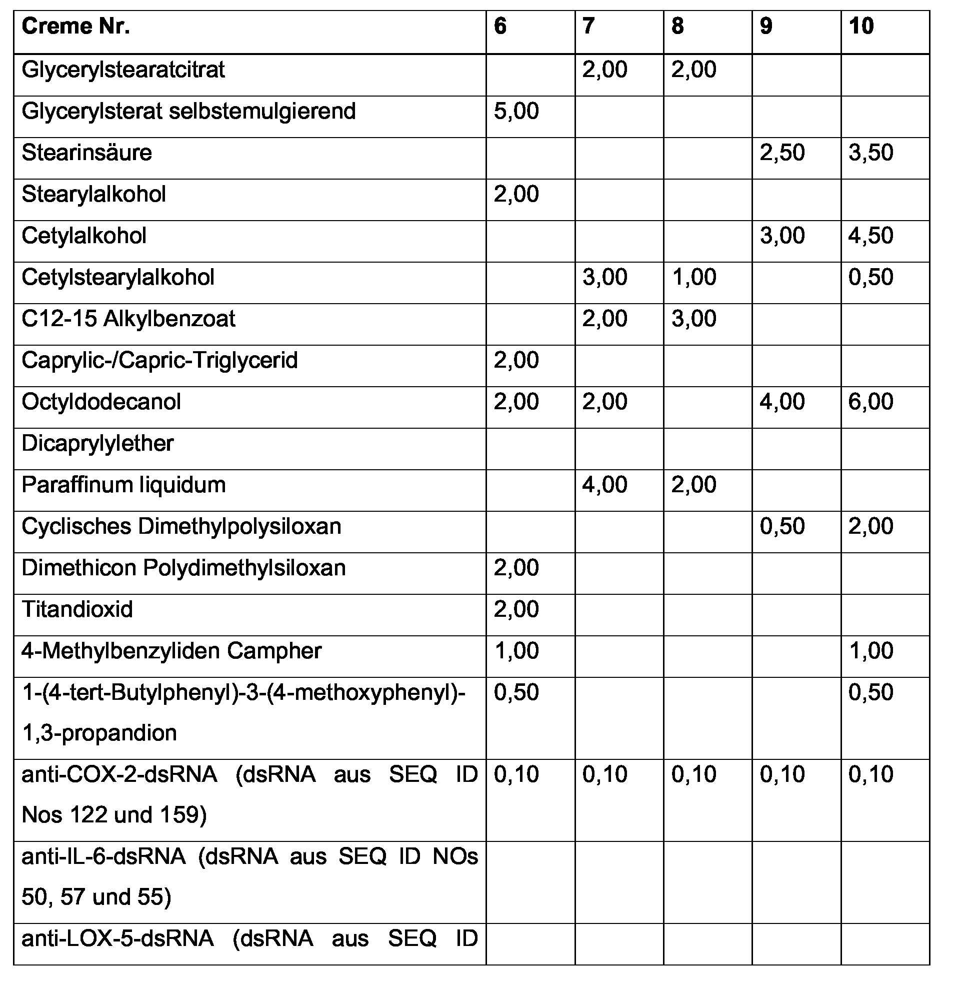

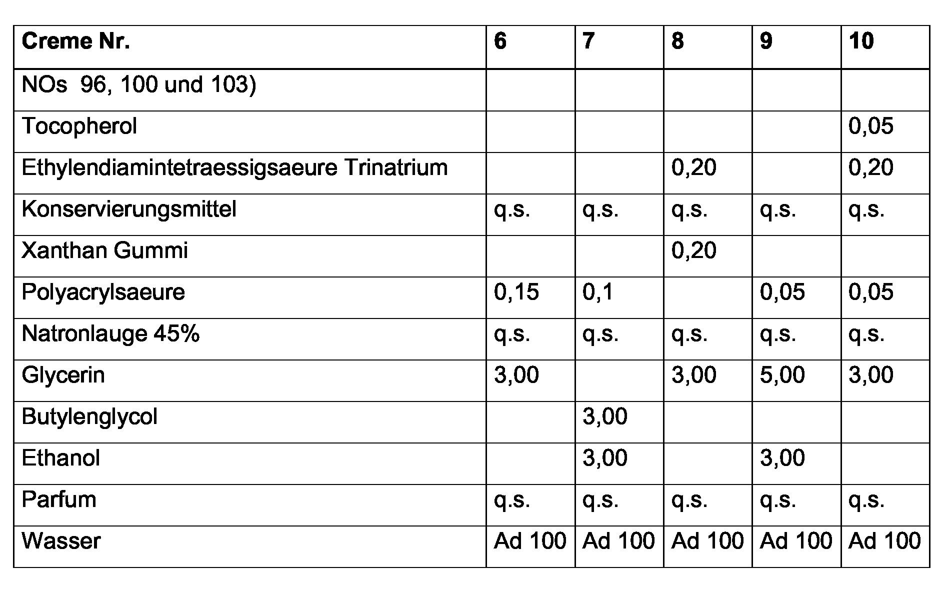

Auf analoge Weise wurde eine Creme unter Verwendung von dsRNA hergestellt, die durch Hybridisieren der Sequenzen SEQ NOs 137 und 172 erhalten wurde. Die Mengenabgaben zu anti-COX-2-dsRNA, anti-IL-6-dsRNA und anti-LOX-5-dsRNA beziehen sich auf die Gesamtmenge an dsRNA, die sich aus den genannten, jeweiligen Sequenzen (SEQ IDs) zusammensetzt.

### Beispiel 3: Herstellung von Wasser-in-Öl-Emulsionen

Durch Mischen der in der Tabelle angegebenen Komponenten wurden Wasser-in-Öl-Emulsionen der ebenfalls angegebenen Zusammensetzung hergestellt. Als Oligoribonukleotid wurde dsRNA verwendet, die durch Hybridisieren des Sense RNA und Antisense RNA Strangs zu SEQ ID NO 183 erhalten wurde. SEQ ID NO 183 ist ein Abschnitt der cDNA der Cyclooxigenase. Die beide Stränge der dsRNA wiesen 3'-ständig jeweils zwei 2'-Desoxythymidinreste auf. Die Mengenabgaben zu anti-COX-2-dsRNA beziehen sich auf die Gesamtmenge an dsRNA, die sich aus den genannten, jeweiligen Sequenzen (SEQ IDs) zusammensetzt.

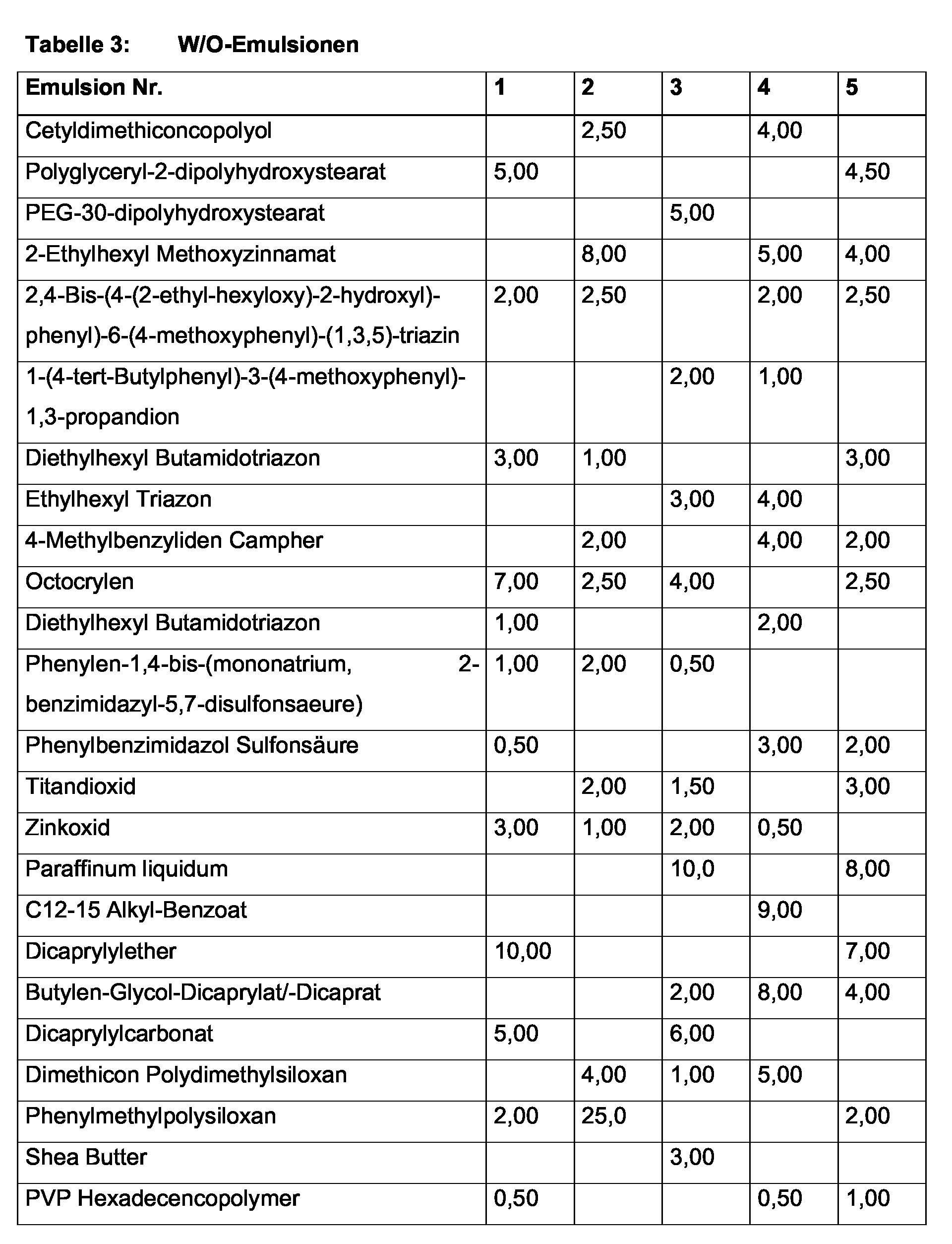

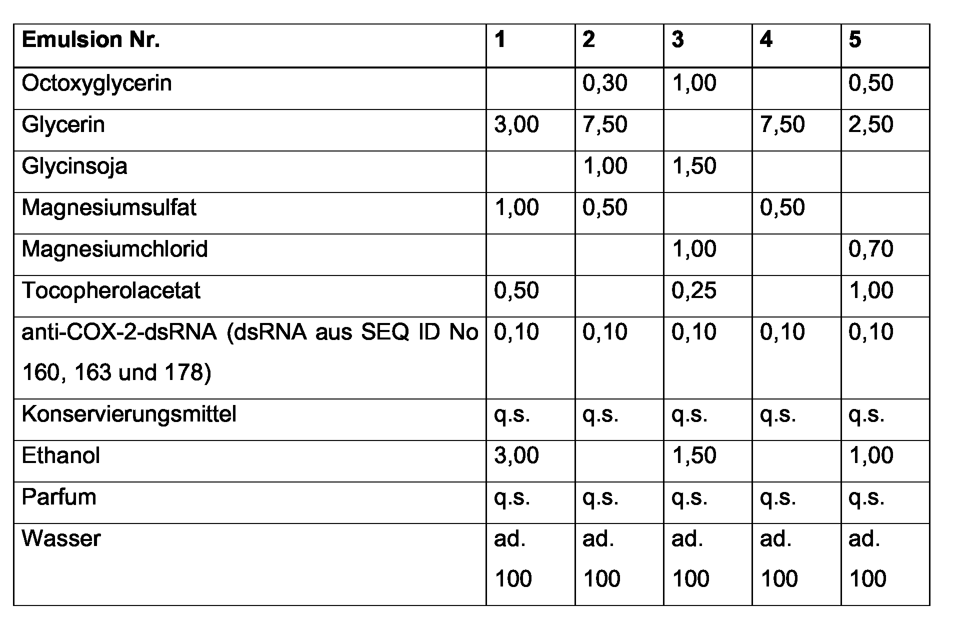

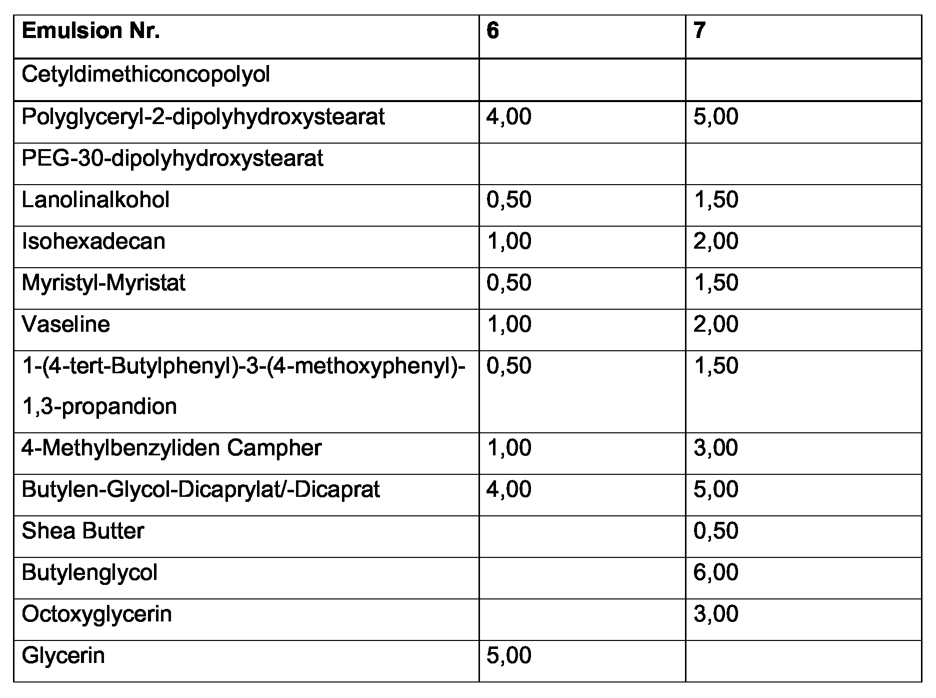

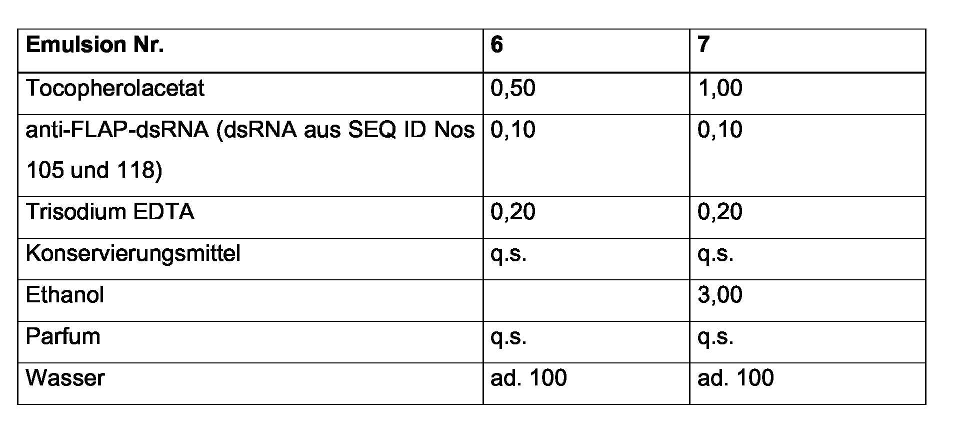

### Beispiel 4: Herstellung von Hydrodispersionen

Durch Mischen der in der Tabelle angegebenen Komponenten wurden Hydrodispersionen der ebenfalls angegebenen Zusammensetzung hergestellt. Als Oligoribonukleotid wurde dsRNA verwendet, die durch Hybridisieren des Sense RNA und Antisense RNA Strangs zu SEQ ID NO 2 und 14 erhalten wurde. SEQ ID NO 2 und 14 sind ein Abschnitt der cDNA des Interleukins-1 alpha. Die beide Stränge der dsRNA wiesen 3'-ständig jeweils zwei 2'-Desoxythymidinreste auf. Die Mengenabgaben zu anti-COX-2-dsRNA, anti-IL-6-dsRNA und anti-LOX-5-dsRNA beziehen sich auf die Gesamtmenge an dsRNA, die sich aus den genannten, jeweiligen Sequenzen (SEQ IDs) zusammensetzt.

**Tabelle 4:**

| **Hydrodispersionen** | | | | | |
|---|---|---|---|---|---|
| **Dispersion Nr** | **1** | **2** | **3** | **4** | **5** |
| Polyoxyethylen(20)cetylstearylether | 1,00 | | | 0,5 | |
| Cetylalkohol | | | 1,00 | | |
| Natriumpolyacrylat | | 0,20 | | 0,30 | |
| Acrylate /C10-30-Alkyl-Acrylat Crosspolymer | 0,50 | | 0,40 | 0,10 | 0,10 |
| Xanthan Gummi | | 0,30 | 0,15 | | 0,50 |
| 2-Ethylhexyl Methoxyzinnamat | | | | 5,00 | 8,00 |
| 2,4-Bis-(4-(2-ethyl-hexyloxy-)2-hydroxyl)- | | 1,50 | | 2,00 | 2,50 |
| phenyl)-6-(4-methoxyphenyl)-(1,3,5)-triazin | | | | | |
| 1-(4-tert-Butylphenyl)-3-(4-methoxyphenyl)-1,3-propandion | 1,00 | | 2,00 | | |
| Diethylhexyl Butamidotriazon | | 2,00 | | 2,00 | 1,00 |
| Ethylhexyl Triazon | 4,00 | | 3,00 | 4,00 | |
| 4-Methylbenzyliden Campher | 4,00 | 4,00 | | | 2,00 |
| Octocrylen | | 4,00 | 4,00 | | 2,50 |
| Phenylen-1,4-bis-(mononatrium, 2-benzimidazyl-5,7-disulfonsaeure | 1,00 | | 0,50 | | 2,00 |
| Phenylbenzimidazol Sulfonsäure | 0,50 | | | 3,00 | |
| Titandioxid | 0,50 | | 2,00 | 3,00 | 1,00 |
| Zinkoxid | 0,50 | 1,00 | 3,00 | | 2,00 |
| C12-15 Alkyl Benzoat | 2,00 | 2,50 | | | |
| Dicaprylylether | | 4,00 | | | |
| Butylenglycol-Dicaprylat/-Dicaprat | 4,00 | | 2,00 | 6,00 | |
| Dicaprylylcarbonat | | 2,00 | 6,00 | | |
| Dimethicon Polydimethylsiloxan | | 0,50 | 1,00 | | |
| Phenylmethylpolysiloxan | 2,00 | | | 0,50 | 2,00 |
| Shea Butter | | 2,00 | | | |
| PVP Hexadecencopolymer | 0,50 | | | 0,50 | 1,00 |
| Octoxyglycerin | | | 1,00 | | 0,50 |
| Glycerin | 3,00 | 7,50 | | 7,50 | 2,50 |
| Glycinsoja | | | 1,50 | | |
| Tocopherolacetat | 0,50 | | 0,25 | | 1,00 |
| anti-IL-1 alpha-dsRNA (dsRNA aus SEQ ID Nos 2 und 14) | 0,10 | 0,10 | 0,10 | 0,10 | 0,10 |
| Konservierungsmittel | q.s. | q.s. | q.s. | q.s. | q.s. |
| Ethanol | 3,00 | 2,00 | 1,50 | | 1,00 |
| Parfum | q.s. | q.s. | q.s. | q.s. | q.s. |
| Wasser | ad.100 | ad. | ad. | ad.100 | ad. |
| | | 100 | 100 | | 100 |

### Beispiel 5: Herstellung einer Gelcreme

Durch Mischen der in der Tabelle angegebenen Komponenten wurde eine Gelcreme der ebenfalls angegebenen Zusammensetzung hergestellt. Der pH-Wert der Gelcreme wurde anschließend auf 6,0 eingestellt.

**Tabelle 5:**

| **Gelcreme** | |
|---|---|
| Acrylat /C10-30 Alkylacrylat Crosspolymer | 0,40 |
| Polyacrylsaeure | 0,20 |
| Xanthan Gummi | 0,10 |
| Cetearylalkohol | 3,00 |
| C12-15 Alkylbenzoat | 4,00 |
| Caprylic/Capric Triglycerid | 3,00 |
| Cyclisches Dimethylpolysiloxan | 5,00 |
| anti-IL-6-dsRNA (dsRNA aus SEQ ID NOs 48 und 51) | 0,10 |
| Glycerin | 3,00 |
| Natriumhydroxid | q.s. |
| Konservierungsmittel | q.s. |
| Parfum | q.s. |
| Wasser | ad 100,0 |
| pH-Wert eingestellt auf 6.0 | |

Auf analoge Weise wurde eine Gelcreme unter Verwendung von dsRNA hergestellt, die durch Hybridisieren der Sequenzen SEQ NOs 53 und 57 erhalten wurde. Die Mengenabgaben zu anti-COX-2-dsRNA, anti-IL-6-dsRNA und anti-LOX-5-dsRNA beziehen sich auf die Gesamtmenge an dsRNA, die sich aus den genannten, jeweiligen Sequenzen (SEQ IDs) zusammensetzt.

### Beispiel 6: Herstellung einer Creme auf der Basis einer Wasser-in-ÖI-Emulsion

Durch Mischen der in der Tabelle angegebenen Komponenten wurde eine Creme der ebenfalls angegebenen Zusammensetzung auf der Basis einer Wasser-in-Öl-Dispersion hergestellt.

**Tabelle 6:**

| **W/O-Creme** | |
|---|---|
| Polyglyceryl-3-Diisostearate | 3,50 |
| Glycerin | 3,00 |
| Polyglyceryl-2-Dipolyhydroxystearate | 3,50 |
| anti-IL-8-dsRNA (dsRNA aus SEQ ID NOs 60 und 66) | 0,10 |
| Konservierungsmittel | q.s. |
| Parfum | q.s. |
| Wasser | ad 100,0 |
| Magnesiumsulfat | 0,6 |
| lsopropylstearat | 2,0 |
| Caprylylether | 8,0 |
| Cetearylisononanoat | 6,0 |

Auf analoge Weise wurde eine Emulsion unter Verwendung von dsRNA hergestellt, die durch Hybridisieren der Sequenzen SEQ NOs 69 und 76 erhalten wurde. Die Mengenabgaben zu anti-COX-2-dsRNA, anti-IL-6-dsRNA und anti-LOX-5-dsRNA beziehen sich auf die Gesamtmenge an dsRNA, die sich aus den genannten, jeweiligen Sequenzen (SEQ IDs) zusammensetzt.

### Beispiel 7: Herstellung einer Creme auf der Basis einer Wasser-in-Öl-in-Wasser Emulsion

Durch Mischen der in der Tabelle angegebenen Komponenten wurde eine Creme der ebenfalls angegebenen Zusammensetzung auf der Basis einer Wasser-in-Öl-in-Wasser-Dispersion hergestellt. Als Oligoribonukleotid wurde dsRNA verwendet, die durch Hybridisieren des Sense RNA und Antisense RNA Strangs zu SEQ ID NO 30 erhalten wurde. Die beide Stränge der dsRNA wiesen 3'-ständig jeweils zwei 2'-Desoxythymidinreste auf.

**Tabelle 7:**

| **W/O/W-Creme** | |
|---|---|
| Glycerylstearat | 3,00 |
| PEG-100-Stearat | 0,75 |
| Behenylalkohol | 2,00 |
| Caprylic-/Capric-Triglycerid | 8,0 |
| Octyldodecanol | 5,00 |
| C12-15 Alkylbenzoat | 3,00 |
| anti-IL-1β-dsRNA (dsRNA aus SEQ ID NO 30) | 0,10 |
| Magnesiumsulfat (MgSO4) | 0,80 |
| Ethylendiamintetraessigsaeure | 0,10 |
| Konservierungsmittel | q.s. |
| Parfum | q.s. |
| Wasser | ad 100,0 |
| pH-Wert eingestellt auf 6.0 | |

SEQUENCE LISTING

<110> Beiersdorf AG, Hamburg

<120> Oligoribonukleotide zur Behandlung entzuendlicher und/oder
irritativer Hauterscheinungen

<130> prtpl 02045

<160> 194

<170> PatentIn version 3.2

<210> 1
<211> 23
<212> DNA
<213> Homo sapiens

<400> 1
aaacctctaa aacatccaag ctt 23

<210> 2
<211> 23
<212> DNA
<213> Homo sapiens

<400> 2
aaggttctga agaagagacg gtt 23

<210> 3
<211> 23
<212> DNA
<213> Homo sapiens

<400> 3
aatacaactt tatgaggatc atc 23

<210> 4
<211> 23
<212> DNA
<213> Homo sapiens

<400> 4
aattcatcct gaatgacgcc ctc 23

<210>  5
<211>  23
<212>  DNA
<213>  Homo sapiens

<400>  5
aagtataatt cgagccaatg atc                                                23


<210>  6
<211>  23
<212>  DNA
<213>  Homo sapiens

<400>  6
aatctggatg aagcagtgaa att                                                23


<210>  7
<211>  23
<212>  DNA
<213>  Homo sapiens

<400>  7
aaattaccgt gattctaaga atc                                                23


<210>  8
<211>  23
<212>  DNA
<213>  Homo sapiens

<400>  8
aactcacggc actaagaact att                                                23


<210>  9
<211>  23
<212>  DNA
<213>  Homo sapiens

<400>  9
aagcaagact actgggtgtg ctt                                                23


<210>  10
<211>  23
<212>  DNA
<213>  Homo sapiens

<400>  10
aaaccaggcg taggtctgga gtc                                              23

<210>  11
<211>  23
<212>  DNA
<213>  Homo sapiens

<400>  11
aagaagctaa atcctttact gtt                                              23

<210>  12
<211>  23
<212>  DNA
<213>  Homo sapiens

<400>  12
aaatccttta ctgttagtca ttt                                              23

<210>  13
<211>  23
<212>  DNA
<213>  Homo sapiens

<400>  13
aaccaacact aacatataat gtt                                              23

<210>  14
<211>  23
<212>  DNA
<213>  Homo sapiens

<400>  14
aatcatttta attattattc ttc                                              23

<210>  15
<211>  23
<212>  DNA
<213>  Homo sapiens

<400>  15
aattattatt cttcataaca att                                              23

<210> 16
<211> 23
<212> DNA
<213> Homo sapiens

<400> 16
aagaaatatg cacaatagca gtt 23

<210> 17
<211> 23
<212> DNA
<213> Homo sapiens

<400> 17
aagaagccac agacctagga ttt 23

<210> 18
<211> 23
<212> DNA
<213> Homo sapiens

<400> 18
aagttgctga tgaactctta atc 23

<210> 19
<211> 23
<212> DNA
<213> Homo sapiens

<400> 19
aagccttcct gccgcaacag ttt 23

<210> 20
<211> 23
<212> DNA
<213> Homo sapiens

<400> 20
aatacttctc gaagccgagc ctc 23

<210> 21
<211> 23
<212> DNA

<213>  Homo sapiens

<400>  21
aaggcaaagc acgaaatgtt att                                                23


<210>  22
<211>  23
<212>  DNA
<213>  Homo sapiens

<400>  22
aatgttattt tttaattatt att                                                23


<210>  23
<211>  23
<212>  DNA
<213>  Homo sapiens

<400>  23
aattattatt tatatatgta ttt                                                23


<210>  24
<211>  23
<212>  DNA
<213>  Homo sapiens

<400>  24
aataaatgta cattaattac ctt                                                23


<210>  25
<211>  23
<212>  DNA
<213>  Homo sapiens

<400>  25
aagggcttca ggcaggccgc gtc                                                23


<210>  26
<211>  23
<212>  DNA
<213>  Homo sapiens

<400>  26
aaaaagcttg gtgatgtctg gtc                                                23

<210>  27
<211>  23
<212>  DNA
<213>  Homo sapiens

<400>  27
aagtggtgtt ctccatgtcc ttt                                                23


<210>  28
<211>  23
<212>  DNA
<213>  Homo sapiens

<400>  28
aatgacaaaa tacctgtggc ctt                                                23


<210>  29
<211>  23
<212>  DNA
<213>  Homo sapiens

<400>  29
aaatacctgt ggccttgggc ctc                                                23


<210>  30
<211>  23
<212>  DNA
<213>  Homo sapiens

<400>  30
aaggaaaaga atctgtacct gtc                                                23


<210>  31
<211>  23
<212>  DNA
<213>  Homo sapiens

<400>  31
aatctgtacc tgtcctgcgt gtt                                                23


<210>  32
<211>  23

<212>  DNA
<213>  Homo sapiens

<400>  32
aagaagaaga tggaaaagcg att                                              23


<210>  33
<211>  23
<212>  DNA
<213>  Homo sapiens

<400>  33
aagatggaaa agcgatttgt ctt                                              23


<210>  34
<211>  23
<212>  DNA
<213>  Homo sapiens

<400>  34
aaatcaataa caagctggaa ttt                                              23


<210>  35
<211>  23
<212>  DNA
<213>  Homo sapiens

<400>  35
aataacaagc tggaatttga gtc                                              23


<210>  36
<211>  23
<212>  DNA
<213>  Homo sapiens

<400>  36
aagcagaaaa catgcccgtc ttc                                              23


<210>  37
<211>  23
<212>  DNA
<213>  Homo sapiens

<400>  37

aagagagctg tacccagaga gtc                                    23

<210>  38
<211>  23
<212>  DNA
<213>  Homo sapiens

<400>  38
aagaaaccct ctgtcattcg ctc                                    23

<210>  39
<211>  23
<212>  DNA
<213>  Homo sapiens

<400>  39
aaccgcttcc ctatttattt att                                    23

<210>  40
<211>  23
<212>  DNA
<213>  Homo sapiens

<400>  40
aatttggact ggtgtgctct ctt                                    23

<210>  41
<211>  23
<212>  DNA
<213>  Homo sapiens

<400>  41
aaaatatata agctcagatt att                                    23

<210>  42
<211>  23
<212>  DNA
<213>  Homo sapiens

<400>  42
aaatatataa gctcagatta ttt                                    23

<210>  43

<211>  23
<212>  DNA
<213>  Homo sapiens

<400>  43
aaatgagcaa atatcatact gtt                                                23


<210>  44
<211>  23
<212>  DNA
<213>  Homo sapiens

<400>  44
aatgagcaaa tatcatactg ttc                                                23


<210>  45
<211>  23
<212>  DNA
<213>  Homo sapiens

<400>  45
aaatatcata ctgttcaatg gtt                                                23


<210>  46
<211>  23
<212>  DNA
<213>  Homo sapiens

<400>  46
aatatcatac tgttcaatgg ttc                                                23


<210>  47
<211>  23
<212>  DNA
<213>  Homo sapiens

<400>  47
aatggttctg aaataaactt ctc                                                23


<210>  48
<211>  23
<212>  DNA
<213>  Homo sapiens

<400> 48
aaagagaagc tctatctccc ctc 23

<210> 49
<211> 23
<212> DNA
<213> Homo sapiens

<400> 49
aactccttct ccacaagcgc ctt 23

<210> 50
<211> 23
<212> DNA
<213> Homo sapiens

<400> 50
aatgggcatt ccttcttctg gtc 23

<210> 51
<211> 23
<212> DNA
<213> Homo sapiens

<400> 51
aaatatgtga agctgagtta att 23

<210> 52
<211> 23
<212> DNA
<213> Homo sapiens

<400> 52
aatatgtgaa gctgagttaa ttt 23

<210> 53
<211> 23
<212> DNA
<213> Homo sapiens

<400> 53
aagaagtacc acttgaaaca ttt 23

<210>  54
<211>  23
<212>  DNA
<213>  Homo sapiens

<400>  54
aatggaaagt ggctatgcag ttt                                          23


<210>  55
<211>  23
<212>  DNA
<213>  Homo sapiens

<400>  55
aaatggctaa ctttatacat att                                          23


<210>  56
<211>  23
<212>  DNA
<213>  Homo sapiens

<400>  56
aatggctaac tttatacata ttt                                          23


<210>  57
<211>  23
<212>  DNA
<213>  Homo sapiens

<400>  57
aaagaaatat ttatattgta ttt                                          23


<210>  58
<211>  23
<212>  DNA
<213>  Homo sapiens

<400>  58
aaaccaccgg aaggaaccat ctc                                          23


<210>  59
<211>  23
<212>  DNA
<213>  Homo sapiens

<400>  59
aacctttcca ccccaaattt atc                                    23

<210>  60
<211>  23
<212>  DNA
<213>  Homo sapiens

<400>  60
aattcataaa aaaattcatt ctc                                    23

<210>  61
<211>  23
<212>  DNA
<213>  Homo sapiens

<400>  61
aaaaattcat tctctgtggt atc                                    23

<210>  62
<211>  23
<212>  DNA
<213>  Homo sapiens

<400>  62
aagcaaatct acttcaacac ttc                                    23

<210>  63
<211>  23
<212>  DNA
<213>  Homo sapiens

<400>  63
aatacaagat tcctggttaa att                                    23

<210>  64
<211>  23
<212>  DNA
<213>  Homo sapiens

<400>  64
aacatactta tatgtaaagt att                                    23

<210> 65
<211> 23
<212> DNA
<213> Homo sapiens

<400> 65
aataattttt aaatataagg att 23


<210> 66
<211> 23
<212> DNA
<213> Homo sapiens

<400> 66
aatttttaaa tataaggatt ttc 23


<210> 67
<211> 23
<212> DNA
<213> Homo sapiens

<400> 67
aagagaatat ccgaacttta att 23


<210> 68
<211> 23
<212> DNA
<213> Homo sapiens

<400> 68
aatttcagga attgaatggg ttt 23


<210> 69
<211> 23
<212> DNA
<213> Homo sapiens

<400> 69
aaaaatgatg ggacaataaa ttt 23


<210> 70
<211> 23
<212> DNA

<213>  Homo sapiens

<400>  70
aaaatgatgg gacaataaat ttt                                              23


<210>  71
<211>  23
<212>  DNA
<213>  Homo sapiens

<400>  71
aaattttgcc ataaagtcaa att                                              23


<210>  72
<211>  23
<212>  DNA
<213>  Homo sapiens

<400>  72
aattttgcca taaagtcaaa ttt                                              23


<210>  73
<211>  23
<212>  DNA
<213>  Homo sapiens

<400>  73
aaagtcaaat ttagctggaa atc                                              23


<210>  74
<211>  23
<212>  DNA
<213>  Homo sapiens

<400>  74
aatttagctg gaaatcctgg att                                              23


<210>  75
<211>  23
<212>  DNA
<213>  Homo sapiens

<400>  75
aagtccttgt tccactgtgc ctt                                              23

<210> 76
<211> 23
<212> DNA
<213> Homo sapiens

<400> 76
aagtgtaact tattaaccta ttt 23


<210> 77
<211> 23
<212> DNA
<213> Homo sapiens

<400> 77
aacttattaa cctatttatt att 23


<210> 78
<211> 23
<212> DNA
<213> Homo sapiens

<400> 78
aacctattta ttatttatgt att 23


<210> 79
<211> 23
<212> DNA
<213> Homo sapiens

<400> 79
aaatagaaga tgaatcattg att 23


<210> 80
<211> 23
<212> DNA
<213> Homo sapiens

<400> 80
aaagatgtta tagtaaattt att 23


<210> 81
<211> 23

<212>  DNA
<213>  Homo sapiens

<400>  81
aagatgttat agtaaattta ttt                                                23


<210>  82
<211>  23
<212>  DNA
<213>  Homo sapiens

<400>  82
aaatttattt tattttagat att                                                23


<210>  83
<211>  23
<212>  DNA
<213>  Homo sapiens

<400>  83
aatgatgttt tattagataa att                                                23


<210>  84
<211>  23
<212>  DNA
<213>  Homo sapiens

<400>  84
aagaaacaat tgggtaccca gtt                                                23


<210>  85
<211>  23
<212>  DNA
<213>  Homo sapiens

<400>  85
aattgggtac ccagttaaat ttt                                                23


<210>  86
<211>  23
<212>  DNA
<213>  Homo sapiens

<400>  86

aagttttaat tgaactaaca atc 23

<210> 87
<211> 23
<212> DNA
<213> Homo sapiens

<400> 87
aattgaacta acaatcctag ttt 23

<210> 88
<211> 23
<212> DNA
<213> Homo sapiens

<400> 88
aactattaaa acagccaaaa ctc 23

<210> 89
<211> 23
<212> DNA
<213> Homo sapiens

<400> 89
aaaacagcca aaactccaca gtc 23

<210> 90
<211> 23
<212> DNA
<213> Homo sapiens

<400> 90
aaatagattc ttataatatt att 23

<210> 91
<211> 23
<212> DNA
<213> Homo sapiens

<400> 91
aatagattct tataatatta ttt 23

<210> 92

<211>  23
<212>  DNA
<213>  Homo sapiens

<400>  92
aatattattt aaatgactgc att                                                23


<210>  93
<211>  23
<212>  DNA
<213>  Homo sapiens

<400>  93
   aatttttttt actgtttctg att                                                 23


<210>  94
<211>  23
<212>  DNA
<213>  Homo sapiens

<400>  94
aaacatttaa aatataattt gtt                                                23


<210>  95
<211>  23
<212>  DNA
<213>  Homo sapiens

<400>  95
aagcacctgc tggacaagcc ctt                                                23


<210>  96
<211>  23
<212>  DNA
<213>  Homo sapiens

<400>  96
aactgggcga gatccagctg gtc                                                23


<210>  97
<211>  23
<212>  DNA
<213>  Homo sapiens

<400>  97
aagcgatgga gaacctgttc atc                                              23


<210>  98
<211>  23
<212>  DNA
<213>  Homo sapiens

<400>  98
aacacatctg gtgtctgagg ttt                                              23


<210>  99
<211>  23
<212>  DNA
<213>  Homo sapiens

<400>  99
aaagaagaca tcccctacta ctt                                              23


<210>  100
<211>  23
<212>  DNA
<213>  Homo sapiens

<400>  100
aagaagacat cccctactac ttc                                              23


<210>  101
<211>  23
<212>  DNA
<213>  Homo sapiens

<400>  101
aagtcctcag gcttccccaa gtc                                              23


<210>  102
<211>  23
<212>  DNA
<213>  Homo sapiens

<400>  102
aagcagctgc catattacta ctt                                              23

<210> 103
<211> 23
<212> DNA
<213> Homo sapiens

<400> 103
aatcacgacc actgatagat gtc 23

<210> 104
<211> 23
<212> DNA
<213> Homo sapiens

<400> 104
aagaaactgt aggcaatgtt gtc 23

<210> 105
<211> 23
<212> DNA
<213> Homo sapiens

<400> 105
aaacgcatca tactcttcct gtt 23

<210> 106
<211> 23
<212> DNA
<213> Homo sapiens

<400> 106
aacgcatcat actcttcctg ttc 23

<210> 107
<211> 23
<212> DNA
<213> Homo sapiens

<400> 107
aactattacc tcatcttctt ttt 23

<210> 108
<211> 23
<212> DNA
<213> Homo sapiens

<400>  108
aagacgatct ccaccaccat ctc                                              23

<210>  109
<211>  23
<212>  DNA
<213>  Homo sapiens

<400>  109
aactctctgc tgaatatggg gtt                                              23

<210>  110
<211>  23
<212>  DNA
<213>  Homo sapiens

<400>  110
aatatggggt tggtgttctc atc                                              23

<210>  111
<211>  23
<212>  DNA
<213>  Homo sapiens

<400>  111
aatacctaca agtcatcata att                                              23

<210>  112
<211>  23
<212>  DNA
<213>  Homo sapiens

<400>  112
aaatctattg gccatctggg ctt                                              23

<210>  113
<211>  23
<212>  DNA
<213>  Homo sapiens

<400>  113
aatctattgg ccatctgggc ttc                                              23

<210> 114
<211> 23
<212> DNA
<213> Homo sapiens

<400> 114
aacatgaccg tggccccaaa ttt 23


<210> 115
<211> 23
<212> DNA
<213> Homo sapiens

<400> 115
aaatttgcta ttcccatgca ttt 23


<210> 116
<211> 23
<212> DNA
<213> Homo sapiens

<400> 116
aatttgctat tcccatgcat ttt 23


<210> 117
<211> 23
<212> DNA
<213> Homo sapiens

<400> 117
aaaataaaat gcagagacat gtt 23


<210> 118
<211> 23
<212> DNA
<213> Homo sapiens

<400> 118
aaataaaatg cagagacatg ttt 23


<210> 119
<211> 23
<212> DNA

<213>  Homo sapiens

<400>  119
aataaaatgc agagacatgt ttt                                    23


<210>  120
<211>  23
<212>  DNA
<213>  Homo sapiens

<400>  120
aactcctcag cagcgcctcc ttc                                    23


<210>  121
<211>  23
<212>  DNA
<213>  Homo sapiens

<400>  121
aaaactgctc aacaccggaa ttt                                    23


<210>  122
<211>  23
<212>  DNA
<213>  Homo sapiens

<400>  122
aactgctcaa caccggaatt ttt                                    23


<210>  123
<211>  23
<212>  DNA
<213>  Homo sapiens

<400>  123
aaaattattt ctgaaaccca ctc                                    23


<210>  124
<211>  23
<212>  DNA
<213>  Homo sapiens

<400>  124
aacgttgtga ataacattcc ctt                                    23

<210> 125
<211> 23
<212> DNA
<213> Homo sapiens

<400> 125
aatgcaatta tgagttatgt gtt 23

<210> 126
<211> 23
<212> DNA
<213> Homo sapiens

<400> 126
aaattgcttc taagaagaaa gtt 23

<210> 127
<211> 23
<212> DNA
<213> Homo sapiens

<400> 127
aattgcttct aagaagaaag ttc 23

<210> 128
<211> 23
<212> DNA
<213> Homo sapiens

<400> 128
aagaagaaag ttcatccctg atc 23

<210> 129
<211> 23
<212> DNA
<213> Homo sapiens

<400> 129
aaatcatatt tacggtgaaa ctc 23

<210> 130
<211> 23

<212>  DNA
<213>  Homo sapiens

<400>  130
aattgatgga gagatgtatc ctc                                              23


<210>  131
<211>  23
<212>  DNA
<213>  Homo sapiens

<400>  131
aagatactca ggcagagatg atc                                              23


<210>  132
<211>  23
<212>  DNA
<213>  Homo sapiens

<400>  132
aagtccctga gcatctacgg ttt                                              23


<210>  133
<211>  23
<212>  DNA
<213>  Homo sapiens

<400>  133
aatggggtga tgagcagttg ttc                                              23


<210>  134
<211>  23
<212>  DNA
<213>  Homo sapiens

<400>  134
aatggggtga tgagcagttg ttc                                              23


<210>  135
<211>  23
<212>  DNA
<213>  Homo sapiens

<400>  135

aatggggtga tgagcagttg ttc 23

<210> 136
<211> 23
<212> DNA
<213> Homo sapiens

<400> 136
aacacttgag tggctatcac ttc 23

<210> 137
<211> 23
<212> DNA
<213> Homo sapiens

<400> 137
aaatttgacc cagaactact ttt 23

<210> 138
<211> 23
<212> DNA
<213> Homo sapiens

<400> 138
aatttgaccc agaactactt ttc 23

<210> 139
<211> 23
<212> DNA
<213> Homo sapiens

<400> 139
aactactttt caacaaacaa ttc 23

<210> 140
<211> 23
<212> DNA
<213> Homo sapiens

<400> 140
aattccagta ccaaaatcgt att 23

<210> 141

<211>  23
<212>  DNA
<213>  Homo sapiens

<400>  141
aaaatcgtat tgctgctgaa ttt                                          23


<210>  142
<211>  23
<212>  DNA
<213>  Homo sapiens

<400>  142
aatacaacta tcaacagttt atc                                          23


<210>  143
<211>  23
<212>  DNA
<213>  Homo sapiens

<400>  143
aacatggaat tacccagttt gtt                                          23


<210>  144
<211>  23
<212>  DNA
<213>  Homo sapiens

<400>  144
aaaagcctcg gccagatgcc atc                                          23


<210>  145
<211>  23
<212>  DNA
<213>  Homo sapiens

<400>  145
aagcctcggc cagatgccat ctt                                          23


<210>  146
<211>  23
<212>  DNA
<213>  Homo sapiens

<400>  146
aaatcatcaa cactgcctca att                                              23


<210>  147
<211>  23
<212>  DNA
<213>  Homo sapiens

<400>  147
aatcatcaac actgcctcaa ttc                                              23


<210>  148
<211>  23
<212>  DNA
<213>  Homo sapiens

<400>  148
aataacgtga agggctgtcc ctt                                              23


<210>  149
<211>  23
<212>  DNA
<213>  Homo sapiens

<400>  149
aagggctgtc cctttacttc att                                              23


<210>  150
<211>  23
<212>  DNA
<213>  Homo sapiens

<400>  150
aacagtcacc atcaatgcaa gtt                                              23


<210>  151
<211>  23
<212>  DNA
<213>  Homo sapiens

<400>  151
aagtctaatg atcatattta ttt                                              23

<210> 152
<211> 23
<212> DNA
<213> Homo sapiens

<400> 152
aatttaatta tttaataata ttt 23


<210> 153
<211> 23
<212> DNA
<213> Homo sapiens

<400> 153
aattatttaa taatatttat att 23


<210> 154
<211> 23
<212> DNA
<213> Homo sapiens

<400> 154
aactccttat gttacttaac atc 23


<210> 155
<211> 23
<212> DNA
<213> Homo sapiens

<400> 155
aagtttggaa aacagttttt att 23


<210> 156
<211> 23
<212> DNA
<213> Homo sapiens

<400> 156
aaagtaaaga tgtttgaata ctt 23


<210> 157
<211> 23
<212> DNA
<213> Homo sapiens

<400> 157
aagatgccaa aatgctgaaa gtt 23

<210> 158
<211> 23
<212> DNA
<213> Homo sapiens

<400> 158
aaagttttta cactgtcgat gtt 23

<210> 159
<211> 23
<212> DNA
<213> Homo sapiens

<400> 159
aagtttttac actgtcgatg ttt 23

<210> 160
<211> 23
<212> DNA
<213> Homo sapiens

<400> 160
aacaaaacag gtatcagtgc att 23

<210> 161
<211> 23
<212> DNA
<213> Homo sapiens

<400> 161
aaaacaggta tcagtgcatt att 23

<210> 162
<211> 23
<212> DNA
<213> Homo sapiens

<400> 162
aatgaatatt taaattagac att 23

<210> 163
<211> 23
<212> DNA
<213> Homo sapiens

<400> 163
aattagacat taccagtaat ttc 23

<210> 164
<211> 23
<212> DNA
<213> Homo sapiens

<400> 164
aaatcagcaa tgaaacaata att 23

<210> 165
<211> 23
<212> DNA
<213> Homo sapiens

<400> 165
aatcagcaat gaaacaataa ttt 23

<210> 166
<211> 23
<212> DNA
<213> Homo sapiens

<400> 166
aatgaaacaa taatttgaaa ttt 23

<210> 167
<211> 23
<212> DNA
<213> Homo sapiens

<400> 167
aataatttga aatttctaaa ttc 23

<210> 168
<211> 23
<212> DNA

<213>  Homo sapiens

<400>  168
aatctgtaaa atcagatgaa att                                    23


<210>  169
<211>  23
<212>  DNA
<213>  Homo sapiens

<400>  169
aaattttact acaattgctt gtt                                    23


<210>  170
<211>  23
<212>  DNA
<213>  Homo sapiens

<400>  170
aaaatatttt ataagtgatg ttc                                    23


<210>  171
<211>  23
<212>  DNA
<213>  Homo sapiens

<400>  171
aagaatatcc tgttgagata ttc                                    23


<210>  172
<211>  23
<212>  DNA
<213>  Homo sapiens

<400>  172
aaaggagaaa agcccaaatt att                                    23


<210>  173
<211>  23
<212>  DNA
<213>  Homo sapiens

<400>  173
aaactttttg aagcaaactt ttt                                    23

<210> 174
<211> 23
<212> DNA
<213> Homo sapiens

<400> 174
aactttttga agcaaacttt ttt 23


<210> 175
<211> 23
<212> DNA
<213> Homo sapiens

<400> 175
aacgatatgt tttctcagat ttt 23


<210> 176
<211> 23
<212> DNA
<213> Homo sapiens

<400> 176
aatttagcag tccatatcac att 23


<210> 177
<211> 23
<212> DNA
<213> Homo sapiens

<400> 177
aatgacctca taaaatacct ctt 23


<210> 178
<211> 23
<212> DNA
<213> Homo sapiens

<400> 178
aaattcattt cacacattaa ttt 23


<210> 179
<211> 23

<212>  DNA
<213>  Homo sapiens

<400>  179
aattcatttc acacattaat ttt                                                23


<210>  180
<211>  23
<212>  DNA
<213>  Homo sapiens

<400>  180
aattcagtag gtgcattgga atc                                                23


<210>  181
<211>  23
<212>  DNA
<213>  Homo sapiens

<400>  181
aaacacttcg tttctcctat ttt                                                23


<210>  182
<211>  23
<212>  DNA
<213>  Homo sapiens

<400>  182
aacctcagct caggactgct att                                                23


<210>  183
<211>  23
<212>  DNA
<213>  Homo sapiens

<400>  183
aagtaactaa tgtttgaaat ttt                                                23


<210>  184
<211>  23
<212>  DNA
<213>  Homo sapiens

<400>  184

aaattagaca ttaccagtaa ttt 23

<210> 185
<211> 23
<212> DNA
<213> Homo sapiens

<400> 185
aaacaaccct cagacgccac atc 23

<210> 186
<211> 23
<212> DNA
<213> Homo sapiens

<400> 186
aagctgccag gcaggttctc ttc 23

<210> 187
<211> 23
<212> DNA
<213> Homo sapiens

<400> 187
aagacagggg ggccccaggg ctc 23

<210> 188
<211> 23
<212> DNA
<213> Homo sapiens

<400> 188
aatcggcccg actatctcga ctt 23

<210> 189
<211> 23
<212> DNA
<213> Homo sapiens

<400> 189
   aatcccttta ttaccccctc ctt 23

<210> 190

<211>  23
<212>  DNA
<213>  Homo sapiens

<400>  190
aagagaattg ggggcttagg gtc                                    23


<210>  191
<211>  23
<212>  DNA
<213>  Homo sapiens

<400>  191
aactcactgg ggcctacagc ttt                                    23


<210>  192
<211>  23
<212>  DNA
<213>  Homo sapiens

<400>  192
aatctggaga ccagggagcc ttt                                    23


<210>  193
<211>  23
<212>  DNA
<213>  Homo sapiens

<400>  193
aatatttatc tgattaagtt gtc                                    23


<210>  194
<211>  23
<212>  DNA
<213>  Homo sapiens

<400>  194
aagttgtcta aacaatgctg att                                    23

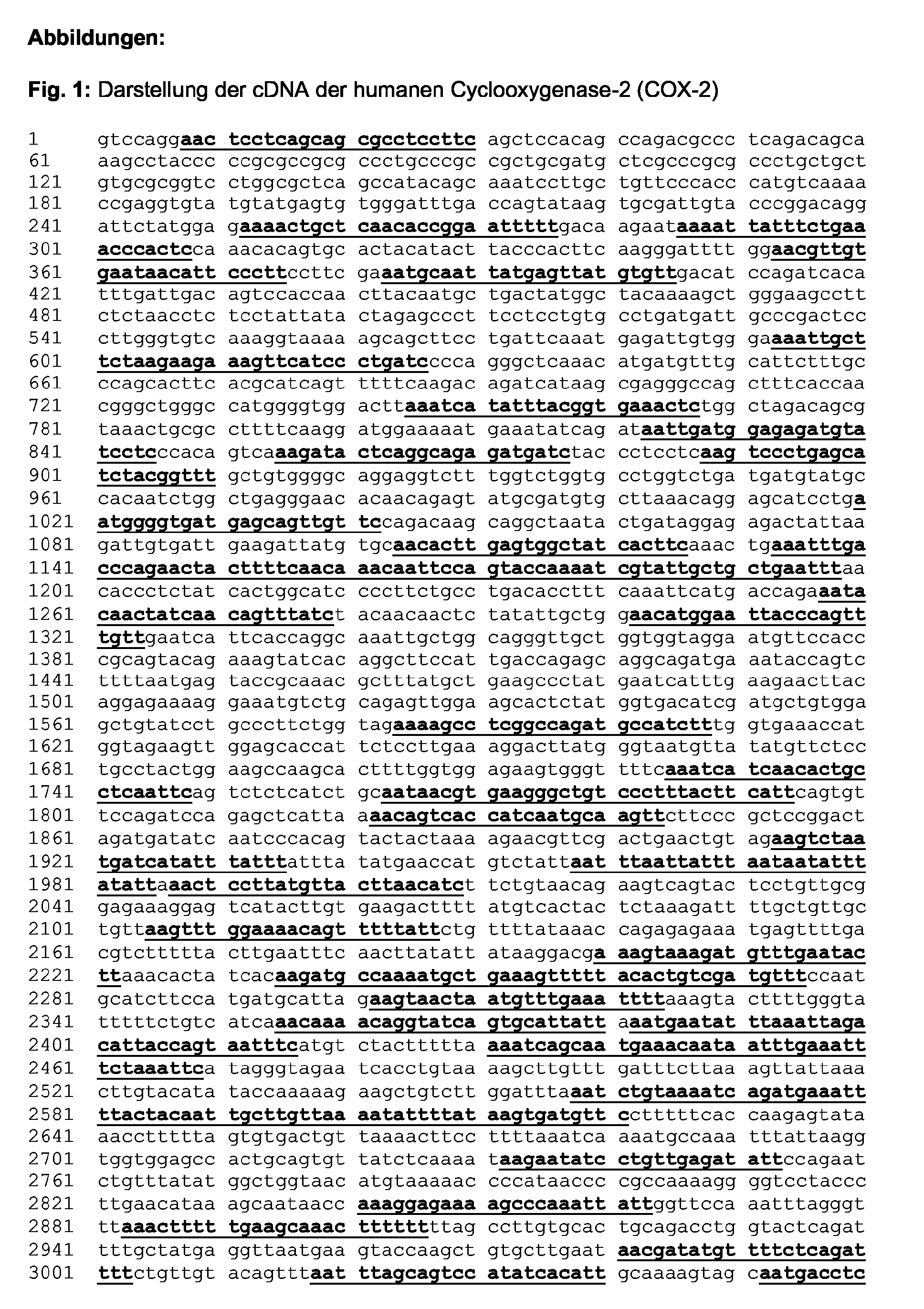

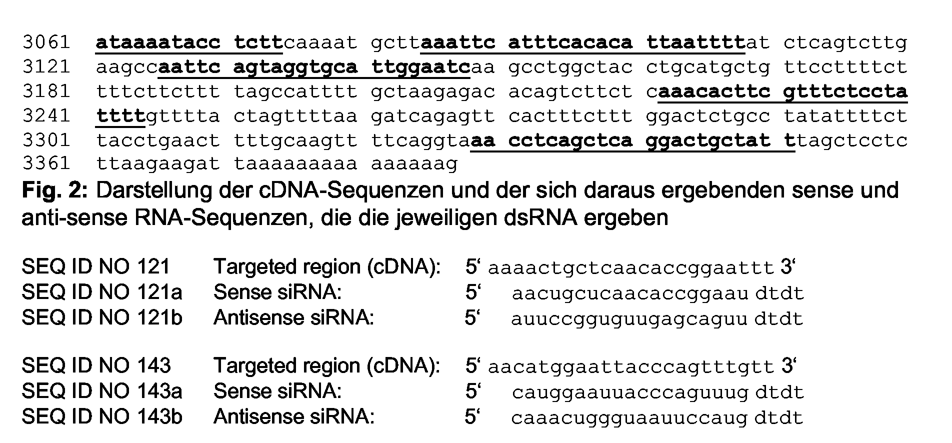

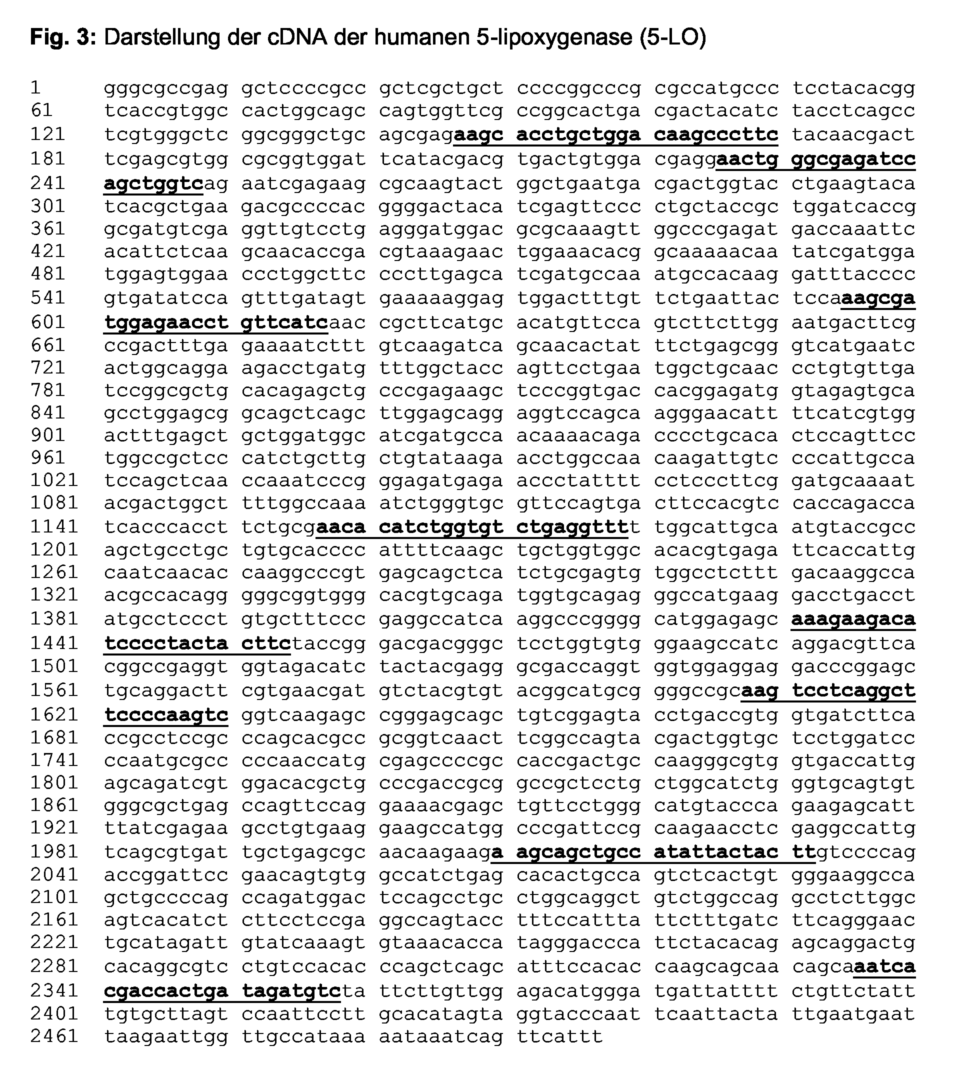

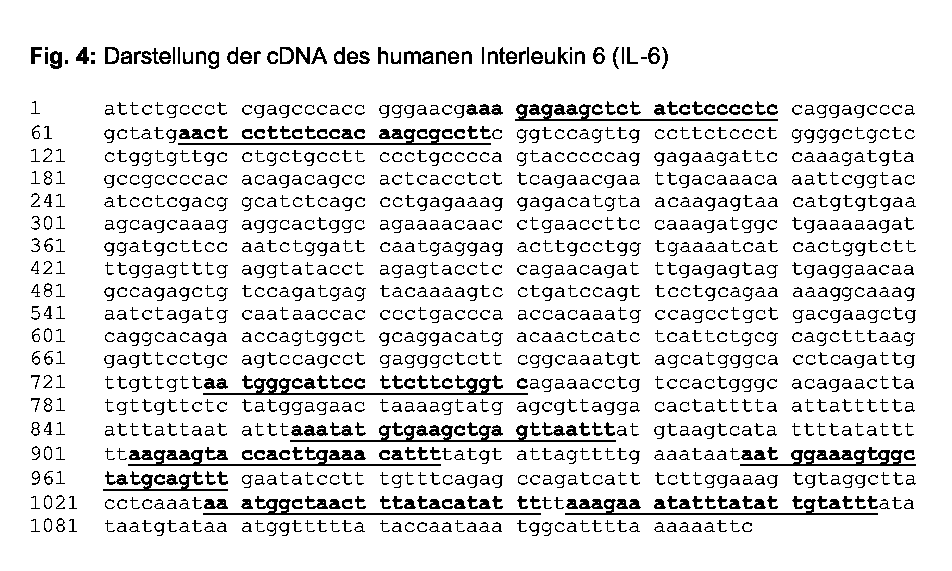

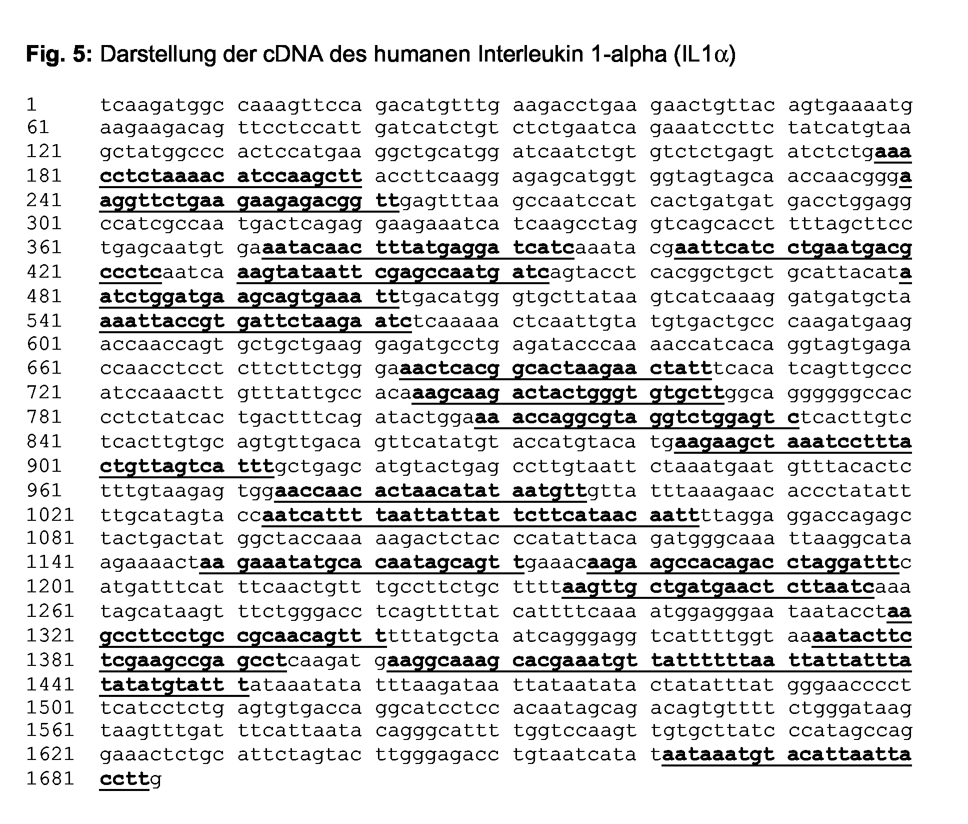

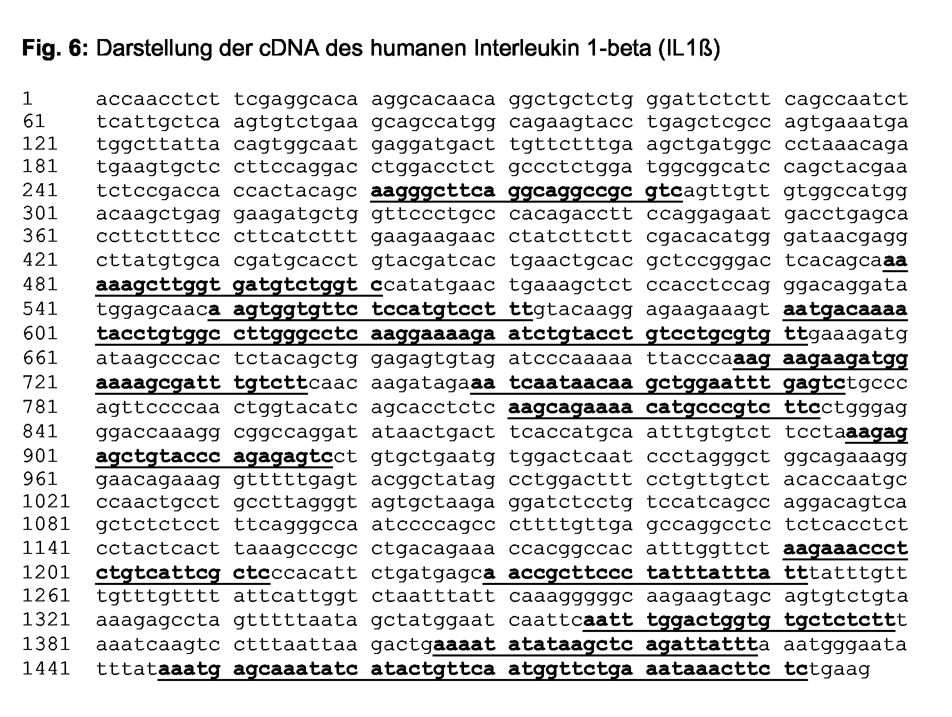

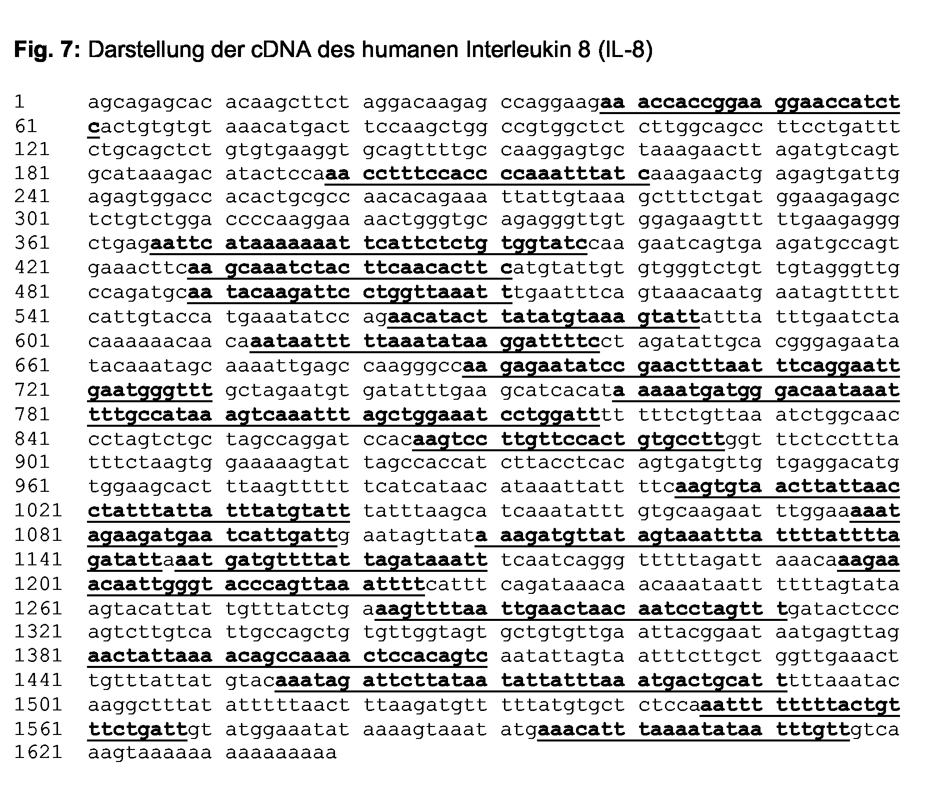

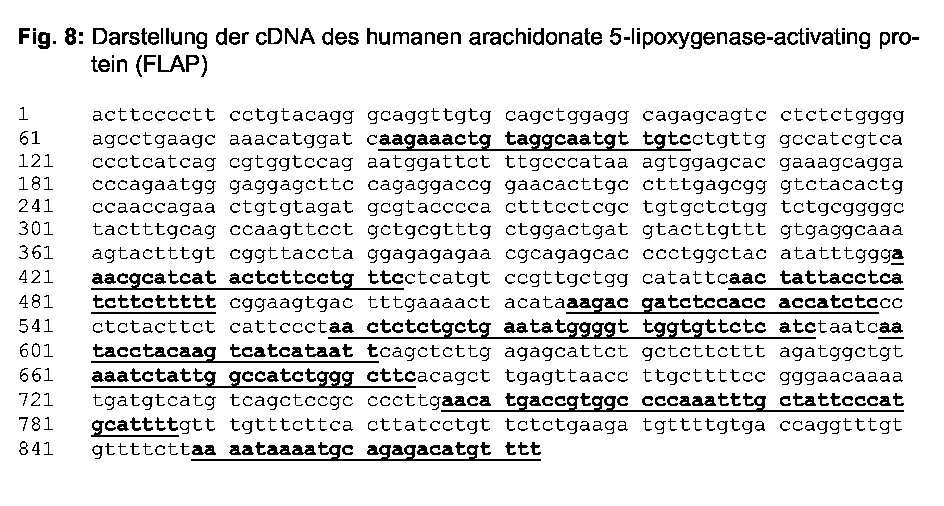

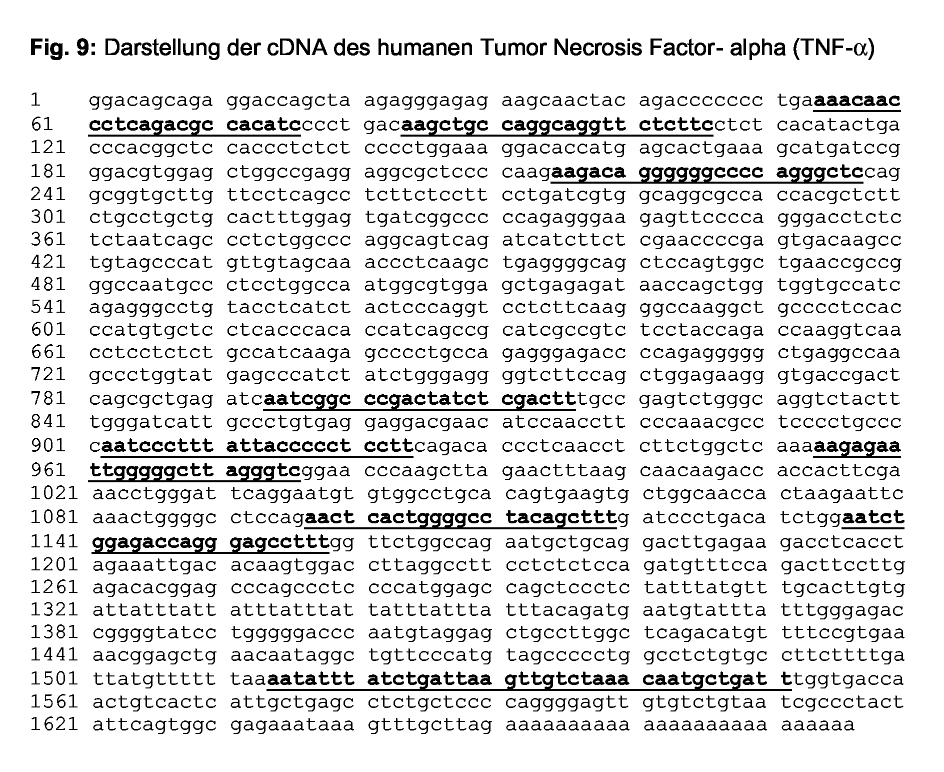

## Patentansprüche

**1.** Doppelsträngiges Oligoribonukleotid oder ein physiologisch verträgliches Salz davon, das in der Lage ist, den Abbau von mRNA von einem oder mehreren an der Entzündung und/oder Irritation der Haut beteiligten Strukturen zu induzieren.

**2.** Oligoribonukleotid nach Anspruch 1, **dadurch gekennzeichnet, dass** die an der Entzündung und/oder Irritation der Haut beteiligten Strukturen an der Produktion von pro-entzündlichen Eicosanoiden oder Cytokinen beteiligt sind.

**3.** Oligoribonukleotid nach Anspruch 2, **dadurch gekennzeichnet, dass** die die an der Entzündung und/oder Irritation der Haut beteiligten Strukturen die Cyclooxygenase 2, die 5-Lipoxygenase oder das 5-Lipoxygenase activating Protein ist.

**4.** Oligoribonukleotid nach Anspruch 2, **dadurch gekennzeichnet**, die an der Entzündung und/oder Irritation der Haut beteiligten Strukturen Interleukin-1alpha oder beta, Interleukin-6, Interleukin-8 oder Tumornekrosefaktor-alpha ist.

**5.** Oligoribonukleotid nach einem der Ansprüche 1 bis 5, **dadurch gekennzeichnet, dass** es die Expression des Gen der an der Entzündung und/oder Irritation der Haut beteiligten Struktur um mindestens 30 % inhibiert.

**7.** Oligoribonukleotid nach Anspruch 6, **dadurch gekennzeichnet, dass** es die Expression des Gen der an der Entzündung und/oder Irritation der Haut beteiligten Struktur um mindestens 50 % inhibiert.

**8.** Oligoribonukleotid nach einem der Ansprüche 1 bis 7, **dadurch gekennzeichnet, dass** es vor der Zielsequenz bezogen auf eine Länge von 20 Basenpaaren in maximal 0 bis 2 Basenpaaren abweicht.

**9.** Oligoribonukleotid nach einem der Ansprüche 1 bis 8, **dadurch gekennzeichnet, dass** es eine Länge von 15 bis 49 Basenpaaren aufweist.

**10.** Oligoribonukleotid nach Anspruch 9, **dadurch gekennzeichnet, dass** eine Länge von 19 bis 25 Basenpaaren aufweist.

**11.** Oligoribonukleotid nach einem der Ansprüche 1 bis 10, **dadurch gekennzeichnet, dass** es homolog zu einem Abschnitt des Gens des der an der Entzündung und/oder Irritation der Haut beteiligten Struktur ist, dessen sense-Strang 5'-seitig durch zwei Adenosinreste und 3'-seitig durch zwei Thymidinreste oder durch einen Thymidin- und einen Cytosinrest flankiert wird.

**12.** Oligoribonukleotid nach einem der Ansprüche 1 bis 11, **dadurch gekennzeichnet, dass** es am 3'-Ende zwei Desoxythymidinreste trägt.

**13.** Oligoribonukleotid nach einem der Ansprüche 1 bis 12, **dadurch gekennzeichnet, dass** es einfach oder mehrfach in einen Expressionsvektor integriert ist.

**14.** Oligoribonukleotid nach einem der Ansprüche 1 bis 13, **dadurch gekennzeichnet, dass** eine oder mehrere Phosphatgruppen durch Phosphothioat-, Methylphosphonat- und/oder Phosphoramidatgruppen ausgetauscht sind.

**15.** Oligoribonukleotid nach einem der Ansprüche 1 bis 14, **dadurch gekennzeichnet, dass** ein oder mehrere Ribosereste durch Aminosäurereste oder Morpholinreste ausgetauscht sind.

**16.** Oligoribonukleotid nach einem der Ansprüche 1 bis 15, **dadurch gekennzeichnet, dass** ein oder mehrere Ribosereste durch Fluor, Alkyl- oder O-Alkylreste modifiziert sind.

**17.** Oligoribonukleotid nach einem der Ansprüche 1 bis 16, **dadurch gekennzeichnet, dass** es ein oder mehrere alpha-Nukleoside enthält.

**18.** Pharmazeutische oder kosmetische Zusammensetzung enthaltend ein oder mehrere Oligoribonukleotide gemäss einem der Ansprüche 1 bis 17 oder ein physiologisch verträgliches Salz davon.

**19.** Zusammensetzung nach Anspruch 18 zur topischen Anwendung.

**20.** Zusammensetzung nach Anspruch 18 oder 19, **dadurch gekennzeichnet, dass** sie mehrere Oligoribonukleotide enthält, die die Expression mehrerer unterschiedlicher an der an der Entzündung und/oder Irritation der Haut beteiligten Strukturen inhibieren.

**21.** Zusammensetzung nach Anspruch 18 oder 19, **dadurch gekennzeichnet, dass** sie mehrere Oligoribonukleotide enthält, die verschiedene Sequenzbereiche ein und desselben Gens der an der Entzündung und/oder Irritation der Haut beteiligten Strukturen zum Ziel haben.

**22.** Zusammensetzung nach einem der Ansprüche 18 bis 21, **dadurch gekennzeichnet, dass** sie 0,00001 bis 10 Gew.-% Oligonukleotid enthält.

**23.** Zusammensetzung nach einem der Ansprüche 18 bis 22, **dadurch gekennzeichnet, dass** sie 1 bis 5 unterschiedliche Oligoribonukleotide enthält.

**24.** Zusammensetzung nach einem der Ansprüche 18 bis 23, **dadurch gekennzeichnet, dass** sie ausschließlich solche Oligoribonukleotide enthält, die die Expression einer oder mehrerer an der Entzündung und/oder Irritation der Haut beteiligten Strukturen inhibieren.

**25.** Zusammensetzung nach einem der Ansprüche 18 bis 25, **dadurch gekennzeichnet, dass** sie in Form einer Lösung, Creme, Salbe, Lotion, Hydrodispersion, Lipodispersion, Emulsion, Pickering-Emulsion, eines Gel, eines festen Stifts oder als Aerosol vorliegt.

**26.** Verwendung eines Oligoribonukleotids gemäss einem der Ansprüche 1 bis 17 oder eines physiologisch verträglichen Salzes davon zur Hautpflege oder kosmetischen oder therapeutischen Behandlung von Entzündungen und/oder Irritationen der Haut.

**27.** Verwendung eines Oligonukleotids gemäss einem der Ansprüche 1 bis 17 oder eines physiologisch verträglichen Salzes davon zur Herstellung einer kosmetischen oder therapeutischen Zusammensetzung zur topischen Applikation.

**28.** Verwendung nach Anspruch 28 zur Herstellung eines kosmetischen oder therapeutischen Mittels zur Hautpflege oder Behandlung von Entzündungen und/oder Irritationen der Haut.
